(19) Europäisches Patentamt
European Patent Office
Office européen des brevets

(11) Publication number: **0 422 638 B1**

(12) # EUROPEAN PATENT SPECIFICATION

(45) Date of publication of patent specification: **18.01.95**

(51) Int. Cl.[6]: **C07H 19/167**, C07H 19/19, C07H 19/056, C07H 19/052, A61K 31/70

(21) Application number: **90119493.6**

(22) Date of filing: **11.10.90**

(54) **Novel inosine/guanosine derivatives.**

(30) Priority: **11.10.89 US 419812**

(43) Date of publication of application:
**17.04.91 Bulletin 91/16**

(45) Publication of the grant of the patent:
**18.01.95 Bulletin 95/03**

(84) Designated Contracting States:
**AT BE CH DE DK ES FR GB GR IT LI LU NL SE**

(56) References cited:
**EP-A- 0 304 889**
**EP-A- 0 334 361**

**JOURNAL OF ORGANIC CHEMISTRY, vol. 44, no. 3, 2nd February 1979, pages 400-456,American Chemical Society; S.D. DIMITRIJEVICH et al.: "Halo sugar nucleosides.6. Synthesis of some 5'-deoxy-5'-iodo and 4',5'-unsaturated purine nucleosides"**

(73) Proprietor: **MERRELL DOW PHARMACEUTICALS INC.**
**2110 East Galbraith Road**
**Cincinnati**
**Ohio 45215 (US)**

(72) Inventor: **Jarvi, Esa T.**
**3953 St.Johns Terrace**
**Cincinnati,**
**Ohio 45236 (US)**
Inventor: **Bitonti, Alan J.**
**7854 Carraway Court**
**Maineville,**
**Ohio 45039 (US)**
Inventor: **McCarthy, James R.**
**6448 Foxview Place**
**West Chester,**
**Ohio 45069 (US)**
Inventor: **Baumann, Russell J.**
**684 Woodyhill Drive**
**Cincinnati,**
**Ohio 45238 (US)**

(74) Representative: **VOSSIUS & PARTNER**
**Postfach 86 07 67**
**D-81634 München (DE)**

**EP 0 422 638 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to certain inosine and guanosine derivatives which are useful as inhibitors of purine nucleoside hydrolases. The compounds of the present invention are useful in the treatment of patients suffering from certain protozoal infections.

Purine nucleoside hydrolases are not found in mammalian cells but are present in various fungi, bacteria and protozoa. These hydrolases in general catalyze the hydrolysis of various ribonucleosides and deoxyribonucleosides to their free bases and respective pentoses. For example, an inosine/guanosine hydrolase, which has been isolated from the protozoa *Trypanosoma cruzi*, the causative agent of Chagas' disease in man, is a nucleoside hydrolase which hydrolyzes inosine to hypoxanthine and ribose, and guanosine to guanine and ribose [See Miller et al., *J.Biol.Chem.* 259, 5073 (1984)]. A 2′-deoxyinosine hydrolase, which has also been isolated from *Trypanosoma cruzi*, hydrolyzes 2′-deoxyinosine to hypoxanthine and 2′-deoxyribose [*Id.*]. Since *T.cruzi* is incapable of *de novo* purine biosynthesis, it is dependent on the salvage pathways, such as via the purine hydrolases, to supply its requirement for the various purine bases. Agents which inhibit the various nucleoside hydrolases therefore would inhibit the growth of these protozoa by depriving them of their source of purine bases. In fact the hypoxanthine analog allopurinol, its ribonucleoside and formycin B, another inosine analog, all exhibit selective antitrypanosomal activity [*Id.*].

An inosine/guanosine hydrolase, as well as another hydrolase which hydrolyses 2′-deoxyinosine and 2′-deoxyguanosine, has also been isolated from *Leishmania donovani*, a human pathogen [Koszalka and Krenitsky, *J.Biol.Chem.* 254, 8185 (1979)]. In addition, an inosine hydrolase has been isolated from *Leishmania tropica (Id.)*.

Inhibition of these purine hydrolases would reasonably be expected to result in antiprotozoal activity. Inhibitors of purine hydrolases would therefore be useful as antiprotozoal agents.

The present invention provides novel inosine/guanosine derivatives of the formula (1)

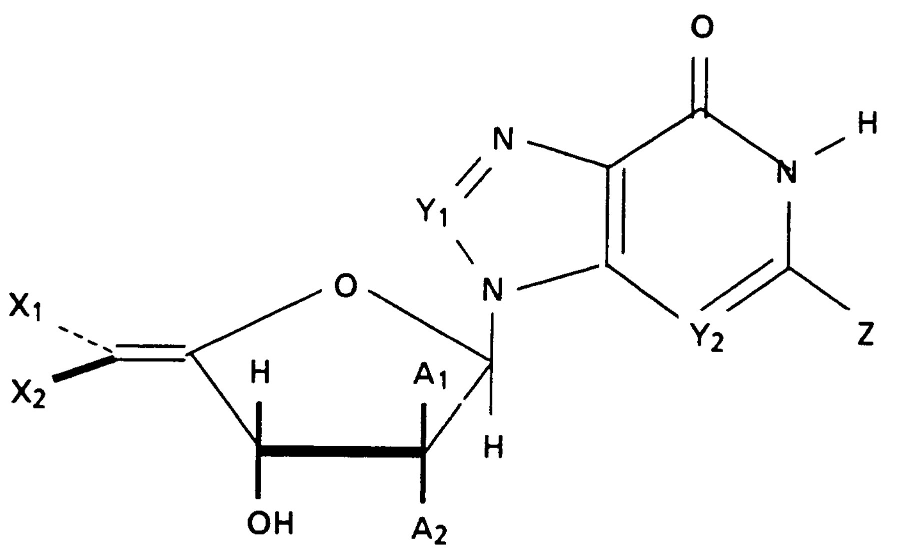

(1)

wherein

- $X_1$ and $X_2$ are each independently hydrogen or halogen with the proviso that at least one of $X_1$ and $X_2$ is a halogen atom,
- $A_1$ and $A_2$ are each independently hydrogen, halogen or hydroxy with the provisos that where $A_1$ is hydroxy, $A_2$ is hydrogen, and where $A_2$ is hydroxy, $A_1$ is hydrogen,
- $Y_1$ is nitrogen, a CH group, a CCl group, a CBr group or a $CNH_2$ group,
- $Y_2$ is nitrogen or a CH group, and
- Z is hydrogen, halogen, or $NH_2$.

The novel compounds of the present invention are inhibitors of purine nucleoside hydrolase, which is present in certain non-mammalian cells such as protozoa.

The present invention also provides a method for treating protozoal infections in a patient suffering therefrom comprising administering to said patient a therapeutically effective antiprotozoal amount of an inosine/guanosine derivative of the present invention.

In addition, the present invention provides a method of inhibiting a purine nucleoside hydrolase in a protozoa comprising contacting said protozoa with an effective purine nucleoside hydrolase inhibitory amount of a compound of formula (1).

As used herein, the terms "halogen" or "halo-" refer to a monovalent fluorine, chlorine, bromine or iodine atom. Of course, it will be appreciated by those skilled in the art that the inosine/guanosine derivatives of the present invention can exist in the keto or enol form. For purposes of convenience, the structures of these inosine/guanosine derivatives are presented in the keto form only. The present invention is not limited to any one form of the inosine/guanosine derivatives and both the keto and enol forms are included within the scope of the invention. It will further be appreciated that the inosine/guanosine derivatives of the present invention can exist in various stereoisomeric forms. The present invention is not limited to any particular stereoisomeric forms and includes all such stereoisomers individually and as racemic mixtures.

The inosine/guanosine derivatives of the present invention can be prepared by utilizing procedures and techniques well known and appreciated by one of ordinary skill in the art.

A general synthetic procedure for making inosine/guanosine derivatives of the formula (1), wherein Z is other than $NH_2$, is set forth in Scheme A. In this scheme the compounds of formula (1) are made from the corresponding 4′-vinylhalo-adenosine derivatives. The term $Z_1$ refers to hydrogen or halogen and all other substituents, unless otherwise indicated, are as previously defined.

## Scheme A

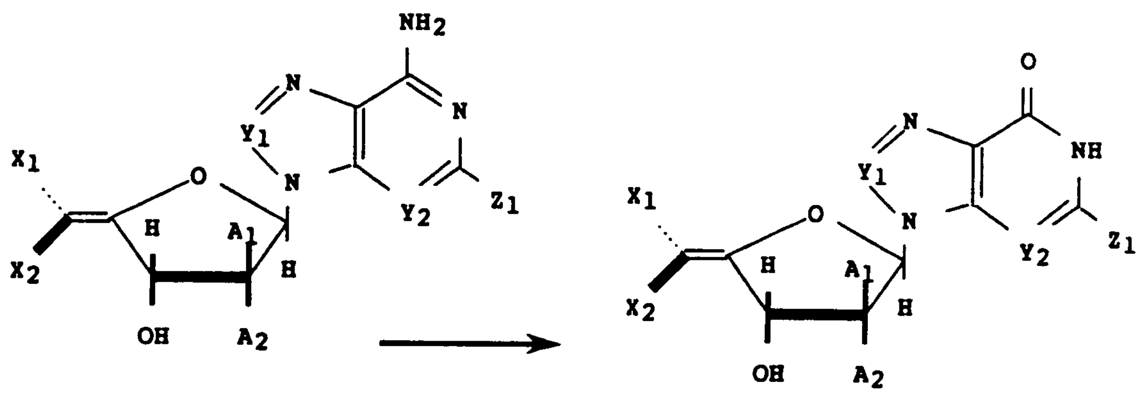

(2) (3)

In this reaction, compounds of the formula (1) are made by subjecting a corresponding 4′-vinylhalo-adenosine derivative to an acidic oxidative deamination.

In Scheme A, the 4′-vinylhalo-adenosine derivative (2) is subjected to an oxidative deamination by means of an acidic oxidative agent to yield the corresponding 4′-vinylhalo-inosine/guanosine derivative (3). The preferred acidic oxidative agent for this reaction is nitrous acid. Where nitrous acid is utilized, the 4′-vinylhalo-adenosine derivative (2) can be dissolved in an organic acid such as acetic acid and sodium nitrate can be added to from the nitrous acid *in situ*. The reaction can generally be carried out at ambient temperature and is completed within several hours.

The following examples present typical syntheses as described by Scheme A. These examples are understood to be illustrative only and are not intended to limit the scope of the present invention in any way. As used herein the following abbreviated terms have the following meanings: mg refers to milligrams, mmol refers to millimoles, mL refers to milliliters, v refers to volume.

EXAMPLE 1

(Z)-5′-Fluoro-4′,5′-didehydro-5′-deoxyinosine

Dissolve (Z)-5′-fluoro-4′,5′-didehydro-5′-deoxyadenosine (267 mg, 1 mmol) in glacial acetic acid (10 mL) with stirring. To this reaction mixture add a solution of sodium nitrite (276 mg, 4 mmol) in water (2 mL). Stir the mixture for 5 hours at ambient temperature. Evaporate the solvent to dryness *in vacuo* and triturate the resulting solid with boiling acetone. Evaporate the acetone extracts to dryness. Recrystallize the title compound from ethanol/water.

EXAMPLE 2

(Z)-5′-Chloro-4′,5′-didehydro-5′-deoxyinosine

Dissolve (Z)-5′-chloro-4′,5′-didehydro-5′-deoxyadenosine (284 mg, 1 mmol) in glacial acetic acid (10 mL) with stirring. To this reaction mixture add a solution of sodium nitrite (276 mg, 4 mmol) in water (2 mL). Stir the mixture for 5 hours at ambient temperature. Evaporate the solvent to dryness *in vacuo* and triturate the resulting solid with boiling acetone. Evaporate the acetone extracts to dryness. Recrystallize the title compound from ethanol/water.

The 4′-vinylhalo-adenosine derivatives (2) which are useful as starting materials in the reaction described in Scheme A, can be prepared by utilizing procedures and techniques which are well known and appreciated by those of ordinary skill in the art.

For example, the 4′-vinylhalo-adenosine derivatives, wherein one of $X_1$ and $X_2$ is hydrogen, can be prepared by a general synthetic procedure as set forth in Scheme B wherein the term "halogen" or "$X_{Hal}$" refers to a fluorine, chlorine, bromine, or iodine atom and the term "nitrogen" refers to a trivalent nitrogen atom attached to two radicals and all other substituents, unless otherwise indicated, are as previously defined.

SCHEME B

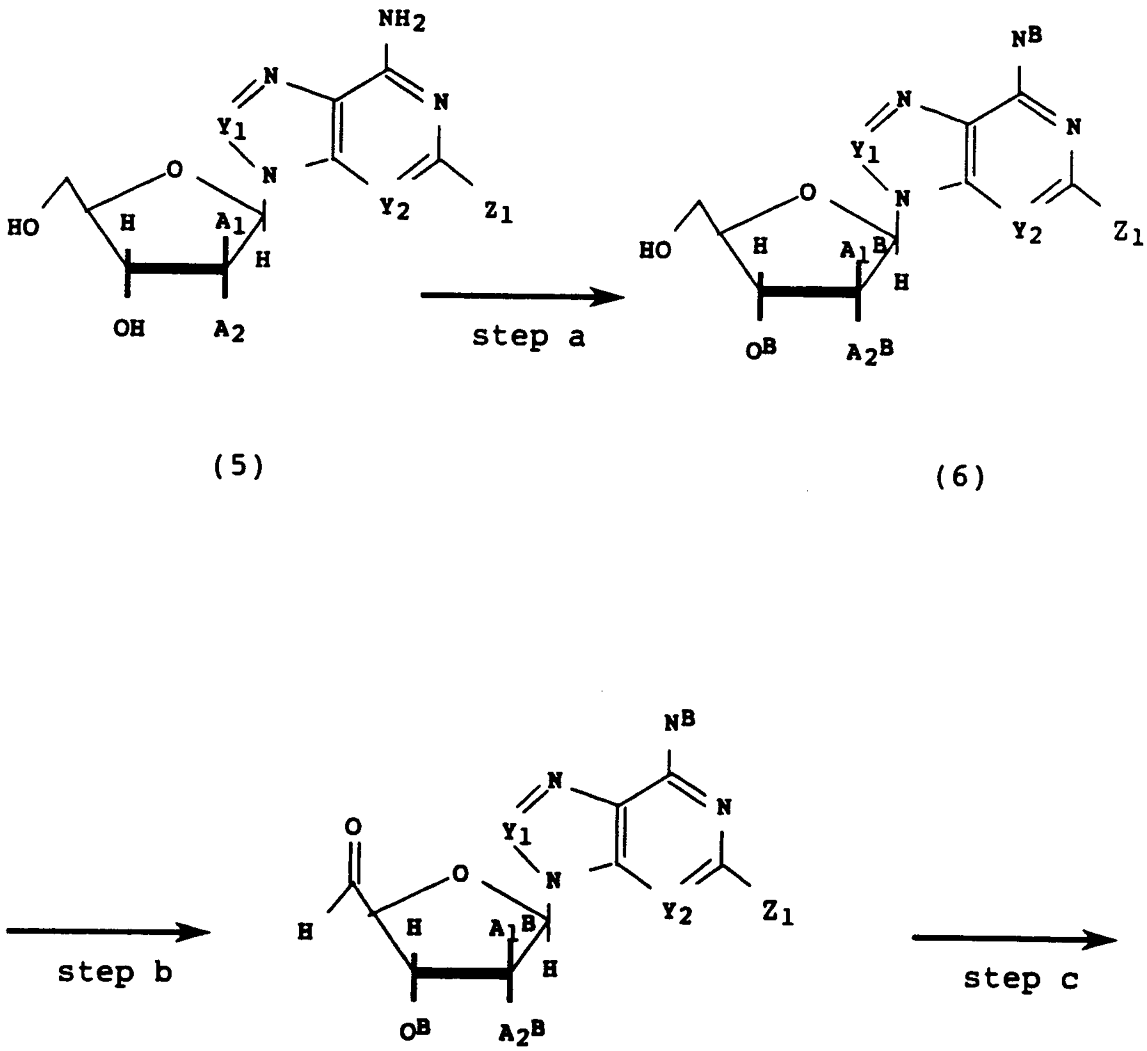


(5)

(6)

(7)

Scheme B (cont'd)

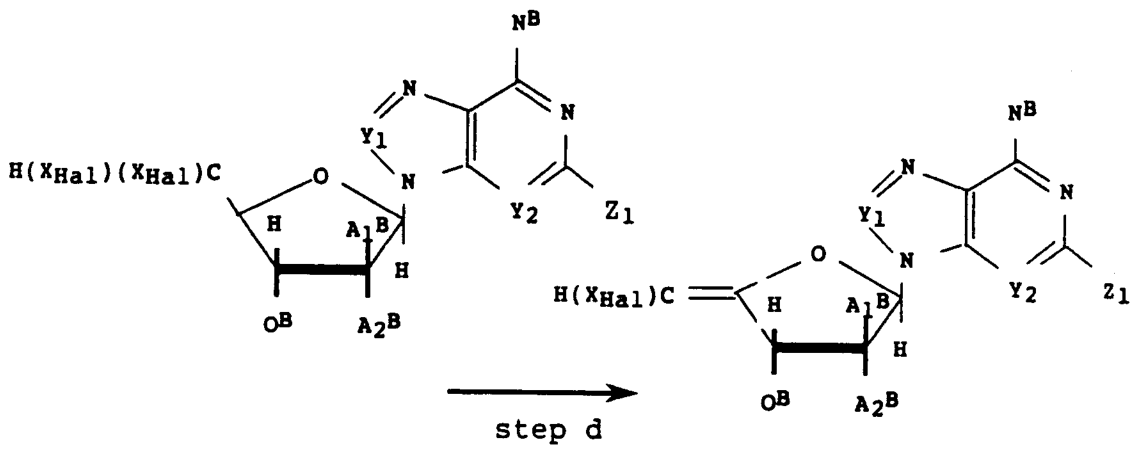


(8)

(9)

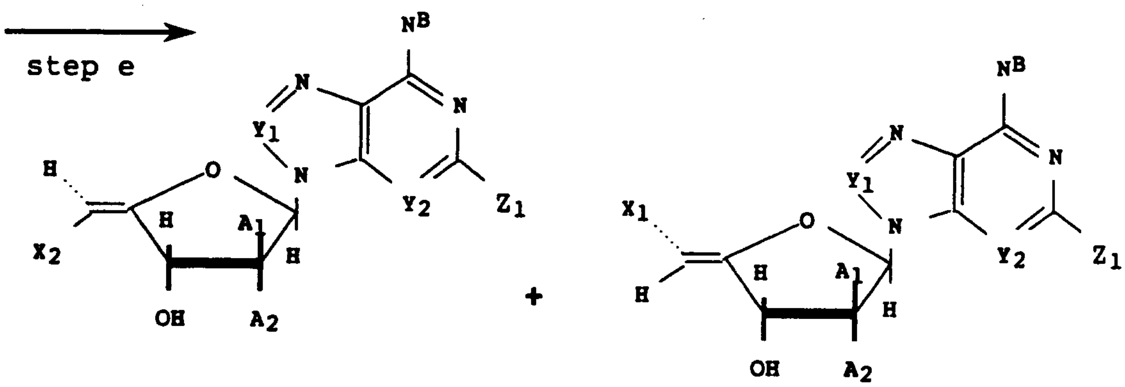


(10)

(11)

## Scheme B (cont'd)

(10) (11)

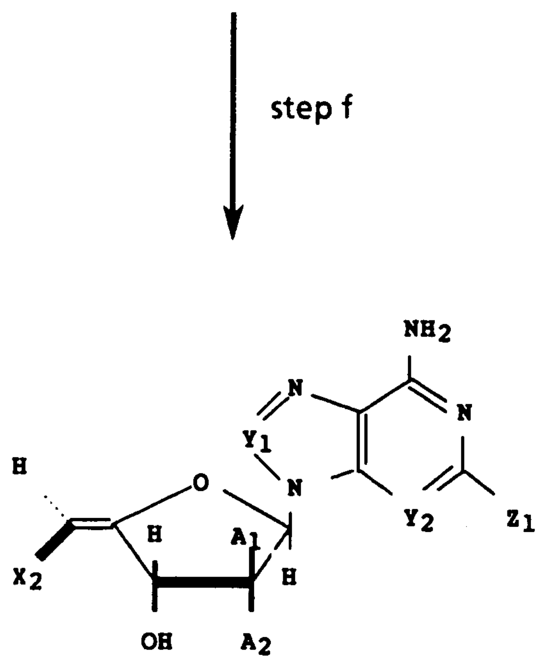


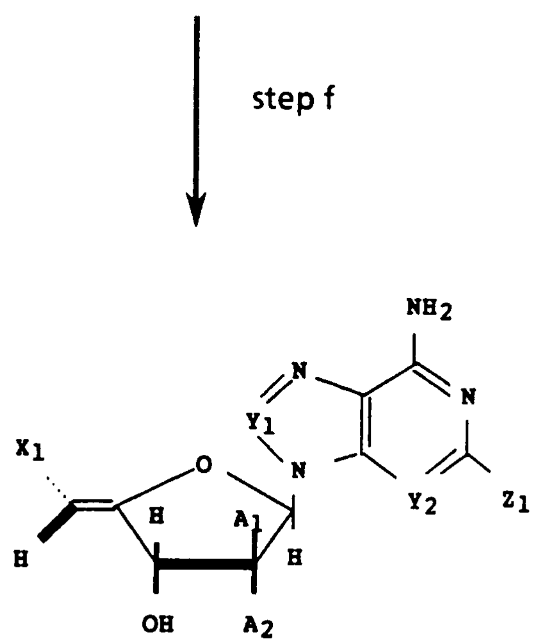


(12) (13)

Basically, in step a, reactive hydroxy or amino groups other than the 5′-hydroxy group are blocked with standard blocking agents well known in the art. These blocking groups can be conventional amino protecting groups for the 6-amino, and conventional hydroxy protecting groups for the 3′-hydroxy and for $A_1$ and $A_2$ (wherein $A_1$ or $A_2$ are OH). $O^B$, $A_1{}^B$, $A_2{}^B$ and $N^B$ in Scheme B represent the 3′-hydroxy, $A_1$, $A_2$ and the 6-amino groups as herein defined blocked with a blocking group where appropriate.

The selection and utilization of particular blocking groups are well known to one of ordinary skill in the art. In general, blocking groups should be selected which adequately protect the amino or hydroxy groups in question during subsequent synthetic steps and which are readily removable under conditions which will not cause degradation of the desired product.

Examples of suitable hydroxy protecting groups are $C_1$-$C_6$ alkyl, tetrahydropyranyl, methoxymethyl, methoxyethoxymethyl, t-butyl, benzoyl, and triphenylmethyl. The term $C_1$-$C_6$ alkyl refers to a saturated hydrocarbyl radical of one to six carbon atoms of straight, branched, or cyclic configuration. The preferred blocking group for the 3′-hydroxy and for $A_2$ (wherein $A_2$ is hydroxy) is 2′,3′-0-isopropylidene. The preferred blocking group for $A_1$ (wherein $A_1$ is hydroxy) and for the 3′-hydroxy (wherein $A_2$ is not hydroxy) is benzoyl. The 2′,3′-0-isopropylidene derivative can be formed by reacting the unblocked compound with acetone. The benzoylated derivative can be formed by reacting the unblocked compound with benzoyl chloride in the presence of pyridine.

Examples of suitable amino protecting groups are benzoyl, formyl, acetyl, trifluoroacetyl, phthalyl, tosyl, benzenesulfonyl, benzyloxycarbonyl, substituted-benzyloxycarbonyl (e.g., p-chloro,p-bromo, p-nitro, p-methoxy, o-chloro, 2,4-dichloro, and 2,6-dichloro derivatives), t-butyloxycarbonyl (Boc), t-amyloxycarbonyl, isopropyloxycarbonyl, 2-(p-biphenyl)-isopropyloxycarbonyl, allyloxycarbonyl, cyclopentyloxycarbonyl, cyclohexyloxycarbonyl, adamantyloxycarbonyl, phenylthiocarbonyl, and triphenylmethyl. Preferred amino

protected compounds include the di-benzoyl derivative, made by reacting the unblocked compound with benzoyl chloride, and the acetyl derivative, made by reacting the unblocked compound with acetic anhydride. It is particularly preferred to protect the 6-amino group as the $N^6,N^6$-di-benzoyl derivative.

In step b, the appropriately blocked 5′-hydroxy derivative (6) is oxidized to the corresponding aldehyde (7). The preferred oxidizing reagent is dicyclohexylcarbodiimide, methyl phosphonic or dichloroacetic acid and dimethylsulfoxide.

The aldehyde (7) can optionally be derivatized so as to improve the handling characteristics of the compound or to facilitate purification thereof by means of procedures and techniques well known and appreciated in the art. For example, the 5′,5′-(N,N′-diphenylethylenediamino) derivative can be prepared by the method of Ranganathan et al. (J. Org. Chem., 39, 290 (1974)].

In step c, the 5′,5′-di-halo derivative (8) is formed by reacting the corresponding aldehyde (7) with a diethylaminosulfur trihalide or similar halo-substituting reagent. Diethylaminosulfur trihalide is preferred.

In step d, the 5′-di-halo derivative (8) is dehydrohalogenated to form the unsaturated (i.e., "(H)($X_{Hal}$)C") derivative (9). The preferred reagent to effect the dehydrohalogenation is potassium t-butoxide in the presence of dimethylsulfoxide.

In step e, the hydroxy protecting groups are removed according to conventional procedures and techniques well known and appreciated in the art. For example, a 2′,3′-0-isopropylidene blocking group can be removed by reacting (9) with aqueous trifluoroacetic acid. The (Z) and (E) isomers, i.e., (10) and (11), respectively, can conveniently be isolated at this stage of the synthesis by the utilization of conventional isolation techniques as are well known and appreciated in the art. Alternatively, the (Z) and (E) isomers can be isolated after deblocking the amino-protecting groups as described below for steps f and g.

In step f, the amino-protecting groups of the (Z) and (E) isomers, i.e., (10) and (11) respectively, are removed utilizing procedures and techniques well known and appreciated in the art. For example, the benzoyl amino blocking groups can be removed by an aminolysis reaction with ammonia.

Starting materials for use in the general synthetic procedure outlined in Scheme B are readily available to one of ordinary skill in the art. For example, certain starting materials for various compounds of formula (1) are listed in Table 1.

TABLE 1

Examples of Starting Materials for *Scheme B*

Compound of formula (1) wherein

| $A_1$ | $A_2$ | $Y_1$ | $Y_2$ | Z | Source of Starting Material |
|---|---|---|---|---|---|
| H | OH | CH | CH | H | J. Med. Chem. 25, 626(1982) |
| OH | H | CH | N | H | Het. Chem. 14, 195 (1977) |
| H | H | CH | N | H | 2′-Deoxyadenosine (commercially available) |
| OH | H | CH | N | F | JACS 86, 1242 (1964) |

Additional starting materials can be prepared by the use of methods analogous to those described in Table 1 as well as other conventional methods as are well known and appreciated in the art.

The following example presents a typical synthesis as described by Scheme B. This example is understood to be illustrative only and is not intended to limit the scope of the present invention in any way.

EXAMPLE 3

(Z) and (E)-4',5'-Didehydro-5'-deoxy-5'-fluoroadenosine

Step a: $N^6$-benzoyl-2',3'-0-isopropylidene-adenosine.

Convert adenosine to its 2',3'-acetonide followed by benzoylation to the $N^6$-benzoyl derivative according to the procedure of Smrt et al. [ Coll. Czech. Chem. Comm. 29, 224 (1964)].

Step b: $N^6$,$N^6$-Bis benzoyl-5-deoxy-2′,3′-0-isopropylidene-5′-,5′-(N,N′-diphenylethylenediamino) adenosine.

Convert $N^6$-benzoyl-2′,3′-0-isopropylidene adenosine to $N^6$-benzoyl-5′-deoxy-2′,3′-0-isopropylidene-5′,5′-(N,N′-diphenylethylenediamino)adenosine according to the procedure of Ranganathan et al. [J. Org. Chem. 39, 290 (1974)]. To 2.96 g of this product in 10 ml of pyridine, cooled in an ice bath, add 1.15 ml (9.9 mmol) of benzoyl chloride. Stir the mixture overnight at room temperature and pour into ice water. Extract the product into 100 ml of chloroform and dry with magnesium sulfate. Evaporate the solution on a rotary evaporator and add toluene. Repeat the evaporation *in vacuo*, and collect 4.07 g of a yellow foam. Percolate the product through a 40 mm X 10 cm flash silica gel column with 4% ethyl acetate/96% dichloromethane. Combine and evaporate the appropriate fractions and collect a yellow oil. Dissolve the oil in ethanol and evaporate three times to yield a solid. Triturate the solid with 50 ml of ethanol and filter. Dry the solid *in vacuo* to give 2.67 g of the title compound [mp 135-138 degrees Celsius (°C)].
NMR ($CDCl_3$, 90 MHz): $\delta$1.30 (3H, S) 1.50 (3H, S), 3.3-3.7 (4H, m), 4.55 (1H, m), 5.1 (2H, d, J = 2), 5.65 (1H, d, J = 2), 6.1 (1H, S), 6.3-7.8 21H, M), 8.40 (1H, S).

Step b continued: $N^6$,$N^6$-Bis benzoyl-2′,3′-0-isopropylidene adenosine-5′-aldehyde.

To 2.64 g (3.73 mmol) of $N^6$,$N^6$-Bis-benzoyl-5′-deoxy-2′,3′-0-isopropylidene-5′,5′-(N,N′-diphenylethylenediamino)adenosine in 370 ml of dichloromethane at 0°C add a solution of 1.56 g (8.2 mmol) p-toluenesulfonic acid monohydrate in 180 ml of acetone. Stir the mixture for 1.5 hours and filter. Evaporate the filtrate on a rotary evaporator and partition the residue between 200 ml of dichloromethane and water. Dry the dichloromethane solution with magnesium sulfate and evaporate to a foam. Dissolve 2.10 g of the foam in 200 ml of benzene and reflux in a Dean-Stark apparatus for one hour. Evaporate the solvent to give 2.06 g of the title compound. (NMR Spectrum reveals more than 80% of the product as aldehyde.)
NMR ($CDCl_3$, 90 MHz): $\delta$ 1.40 (3H, S) 1.70 (3H, S), 4.65 (1H, S), 5.3 (1H, d, J = 7), 5.45 (1H, broad d, J = 7), 6.2 (1H, S), 7.2-7.8 (10H, m), 8.10 (1H, S), 8.45 (major) and 8.55 (1H together, two S). 9.3 (1H, S, CHO).

Step c: $N^6$,$N^6$-Bis-benzoyl-5′-deoxy-5′,5′-difluoro-2′,3′-0-isopropylideneadenosine.

Chromatograph 6.5 g of $N^6$,$N^6$-bis-benzoyl-2′,3′-0-isopropylideneadenosine-5′-aldehyde on a 40 mm x 7 cm flash silica gel column with 15% ethyl acetate/85% dichloromethane solvent. Combine and evaporate all fractions with UV - active material on Thin Layer Chromatography (TLC) to give 5.2 g of a foam. Reflux the foam in 200 ml of benzene for 2 hours and then evaporate and dry *in vacuo* to give 4.65 g of purified $N^6$,$N^6$-bis-benzoyl-2′3′-0-isopropylideneadenosine-5′-aldehyde. Dissolve 3.90 g of the 5′-aldehyde in 25 ml of dichloromethane (distilled from calcium hydride) and to this solution add 3.2 ml (3 equivalents) of diethylaminosulfur trifluoride. Stir the mixture for 6 hours. Dilute the mixture with chloroform and pour into 50 ml of stirred saturated aqueous sodium bicarbonate. Extract the product into 400 ml of chloroform and dry with $MgSO_4$. Evaporate the solvent to give 3.60 g of a foam. Percolate the product through a 40 mm x 12 cm silica gel flash column with 4% ethyl acetate/96% dichloromethane solvent. Isolate the title compound (738 mg) by TLC ($R_f$ 0.6 with 10% ethyl acetate/90% dichloromethane as solvent).
NMR ($CDCl_3$, 300 MHz): $\delta$ 1.42 (3H, S) 1.65 (3H, S) 4.42-4.53 (1H, three m), 5.27 (1H, dd, J = 2.7, 5.9), 5.39 (1H, dd, J = 1.7, 6.0), 5.96 (1H, td, J = 55, 4.5), 7.34- 7.52 (6H, m), 7.85 (4H, d J = 7.2), 8.15 (1H, S), 8.67 (1H, S).
19F-NMR ($CDCl_3$, 282 MHz, ppm from external $CFCl_3$)- 54.87 (ddd, J = 12.4, 55.2, 299.0) - 50.71 (ddd, J = 10, 55.2, 299.1)
MS (FAB - XENON) M + 1 = 536

| | | |
|---|---|---|
| Anal: Calc'd for $C_{27}H_{23}F_2N_5O_5$. | C 60.56, | H 4.33 |
| Found: | C 60.26, | H 4.44 |

Step d: $N^6$ Benzoyl-4′,5′-didehydro-2′,3′-0-isopropylidene-5′-deoxy-5′-fluoroadenosine

To 401 mg (0.75 mmol) of crushed $N^6$,$N^6$-Bis-benzoyl-5′-deoxy-5′,5′-difluoro-2′,3′-0-isopropylideneadenosine and 335 mg (4 equivalents) of potassium t-butoxide under nitrogen add 2 ml of dimethylsulfoxide (distilled from calcium hydride). Stir the mixture under nitrogen for 21 hours. Quench with 4 ml of saturated ammonium chloride and extract with ethyl acetate to yield 274 mg of yellow oil. Percolate the oil through a 20 mm x 15 cm flash column with 30% ethyl acetate/70% dichloromethane. Combine fractions that have two spots close together at Rf = 0.55 (TLC with ethyl acetate as solvent. Evaporate these fractions to yield 183 mg of the title compound containing two isomers in a 2:1 ratio.
NMR ($CDCl_3$, 300 MHz): δ 1.34 and 1.37 (minor) 3H together two S.), 1.49 (3H, s), 5.35-5.38 (1H, m), 5.56 and 5.90 (1H together; d, J = 4 and m, resp.), 6.23 (broad s, minor) and 6.25 (1H together), 6.43 (d, J = 74, major) and 6.81 (d, J = 77; 1H together), 7.39-7.98 (6H, m), 8.646 (major) and 8.653 (minor; two s, 1H together), 9.05 (1H, broad, NH)
NMR $^{19}F$, 282 MHz, ppm from external $CFCl_3$): δ- 158.94 (d, J = 74 major), 174.4 (d, J = 77, minor) MS: (CI) M + 1 = 412.

Step e: $N^6$-Benzoyl-4′,5′-didehydro-5′-deoxy-5′-fluoro adenosine

Dissolve 178 mg of $N^6$-benzoyl-4′,5′-didehydro-2′,3′-0-isopropylidene-5′-deoxy-5′-fluoroadenosine (2:1 mixture of isomers) in 2 ml of cold trifluoroacetic acid-water (4:1). Stir the mixture at room temperature for 50 minutes and then evaporate on a rotary evaporator. Chromatograph the residue on a 20 mm x 14 cm flash silica gel column with ethyl acetate as the solvent. Combine fractions to give 3 mg of the higher $R_f$ isomer (minor isomer), 58 mg of a mixture of isomers and 83 mg of the lower $R_f$ isomer (major isomer) of the title compound.
NMR ($CD_3OD$, higher $R_f$ isomer, 90 MHz): δ 5.1 (2H, m), 6.35 (1H, d, J = 6), (1H, D, J = 74), 7.5-8.2 (5H, m), 8.63 (1H, s), 8.72 (1H, S).
NMR ($CD_3OD$, major lower $R_f$ isomer, 90 MHz): δ 5.00-5.10 (2H, m), 6.37 (1H, d, J = 7), 6.48 (1H, a, J = 75), 7.54-8.19 (5H, m), 8.53 (1H, s), 8.62 (1H, s).

Step f: (Z)-4′,5′-didehydro-5′-deoxy-5′-fluoroadenosine.

Dissolve 83 mg of $N^6$-benzoyl-4′,5′-didehydro-5′-deoxy-5′-fluoroadenosine (lower $R_f$ isomer above) in absolute ethanol, evaporate and redissolve in 6 ml of ethanol. Bubble anhydrous ammonia through the ice cooled solution in a 20 mm x 12 cm Carius tube. Seal the tube and remove the ice bath. After 14 hours at room temperature, open the tube and evaporate the solution to give 87 mg of crude product. Triturate in 1 ml of methanol and filter off the solid. Dry the product *in vacuo* to give 20 mg of the title compound (a white powder, softens at 100-110 °C and melts at 225-230 °C).
NMR ($CD_3OD$, 300 MHz): δ 5.02-5.05 (2H, m), 6.28 (1H, d, J = F), 6.56 (1H, d, J = 7.52), 8.21 (1H, s), 8.33 (1H, s).
$^{19}F$-NMR (282 MHz, ppm from external $CFCl_3$): -166.76 (d, J = 75.2)
MS: (FAB-XENON) M + 1 = 268

Step f: 4′,5′-didehydro-5′-deoxy-5′-fluoroadenosine,with E-isomer as major component.

Dissolve 58 mg of $N^6$-benzoyl-4′,5′-didehydro-5′-deoxy-5′-fluoroadenosine (a mixture with the higher $R_f$ isomer being the major isomer) in 5 ml of absolute ethanol, and bubble ammonia through the ice cooled solution in a 20 mm x 12 cm Carius tube for 3 three minutes. Seal the tube and remove the ice bath. After 15 hours at room temperature, open the tube and evaporate the solution. Dissolve the residue in 2 ml of methanol and chromatograph on a 20 mm x 12 cm silica gel flash column. Eluted with ethyl acetate, followed by 10% methanol/90% ethyl acetate. Combine and evaporate fractions containing material at $R_f$ 0.23 (10% methanol/90% ethyl acetate) to yield 30 mg of product. Triturated in 12 mg of methanol and filter off the solid. Dry the product *in vacuo* to yield 16 mg of the title compound (an off-white powder). NMR indicates a 4:1 mixture of E-isomer to Z-isomer.

$^1$H-NMR (E-isomer $CD_3OD$ 300 MHz): δ5.03-5.07 (2H, m) 6.21 (1H, d, J = 6.3), 7.02 (1H, d, J = 78.6), 8.20 (1H, s), 8.32 (1H, s).
$^{19}$F-NMR (E-isomer, $CD_3OD$, 282 MHz, ppm from ext. $CFCl_3$): - 182.30 (d, J = 78.5).
MS: (CI) mH + = 268.

The following specific compounds can be made by procedures analogous to those described above in Example 3:

(E) and (Z)-5′-Bromo-4′,5′-didehydro-5′-deoxyadenosine
(E) and (Z)-5′-Chloro-4′,5′-didehydro-5′-deoxyadenosine
(E) and (Z)-5′-Bromo-4′,5′-didehydro-2′,5′-dideoxyadenosine
(E) and (Z)-5′-Chloro-4′,5′-didehydro-2′,5′-dideoxyadenosine
(E) and (Z)-5′-Fluoro-4′,5′-didehydro-2′,5′-dideoxyadenosine.

These adenosine derivatives can then be converted to the appropriate inosine/guanosine derivatives of formula (1) by the utilization of the procedure as described in Scheme A to yield the following specific compounds:

(E) and (Z)-5′-Bromo-4′,5′-didehydro-5′-deoxyinosine
(E) and (Z)-5′-Chloro-4′,5′-didehydro-5′-deoxyinosine
(E) and (Z)-5′-Fluoro-4′,5′-didehydro-5′-deoxyinosine
(E) and (Z)-5′-Bromo-4′,5′-didehydro-2′,5′-dideoxyinosine
(E) and (Z)-5′-Chloro-4′,5′-didehydro-2′,5′-dideoxyinosine
(E) and (Z)-5′-Fluoro-4′,5′-didehydro-2′,5′-dideoxyinosine

The 4′-vinylhalo-inosine/guanosine derivatives of the formula (1), wherein one of $X_1$ and $X_2$ is hydrogen and the other is halogen, can alternately be prepared by a procedure as described in Scheme C, wherein all terms are as previously defined and the term "4-MeO-ϕ-" refers to a 4-methoxyphenyl group.

SCHEME C

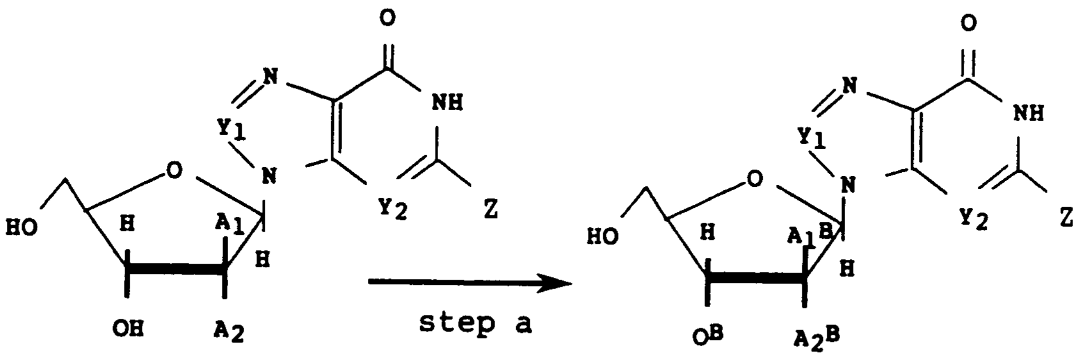


(14) (15)

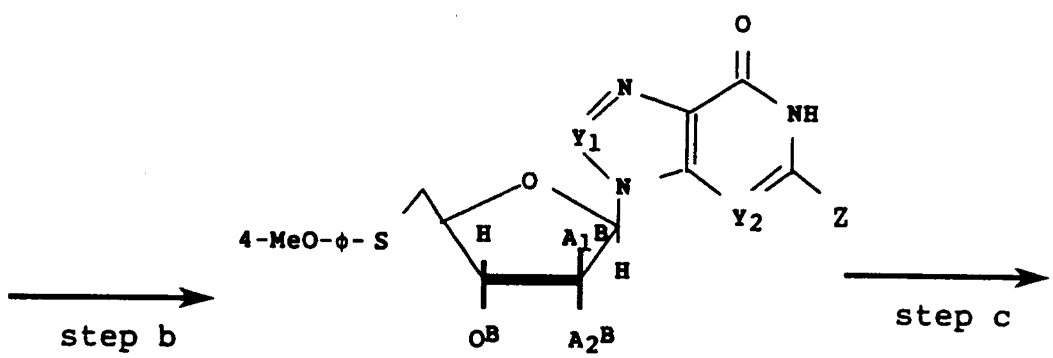


(16)

Scheme C (cont'd)

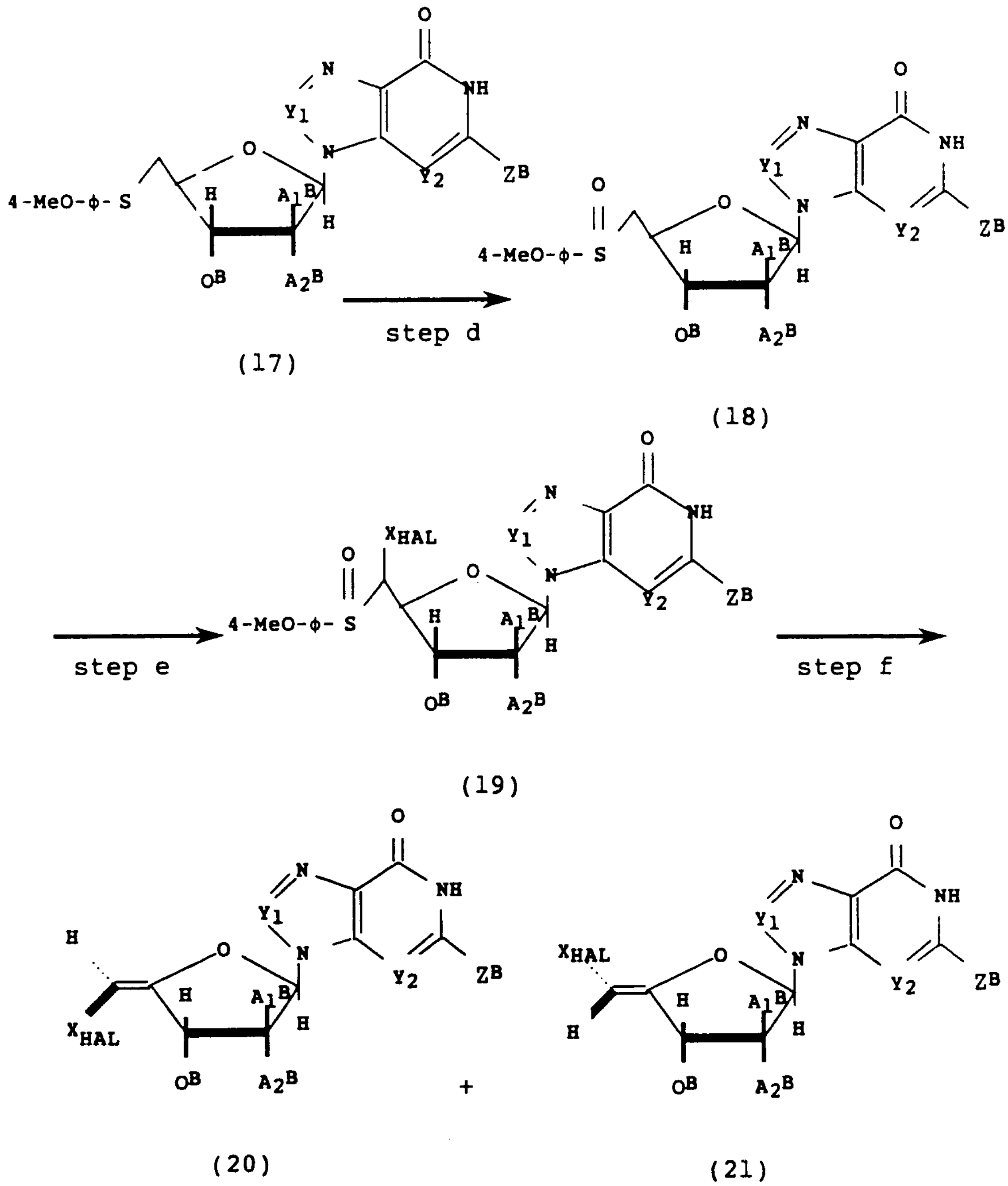


(17)

(18)

(19)

(20) + (21)

Scheme C (cont'd)

(20) (21)

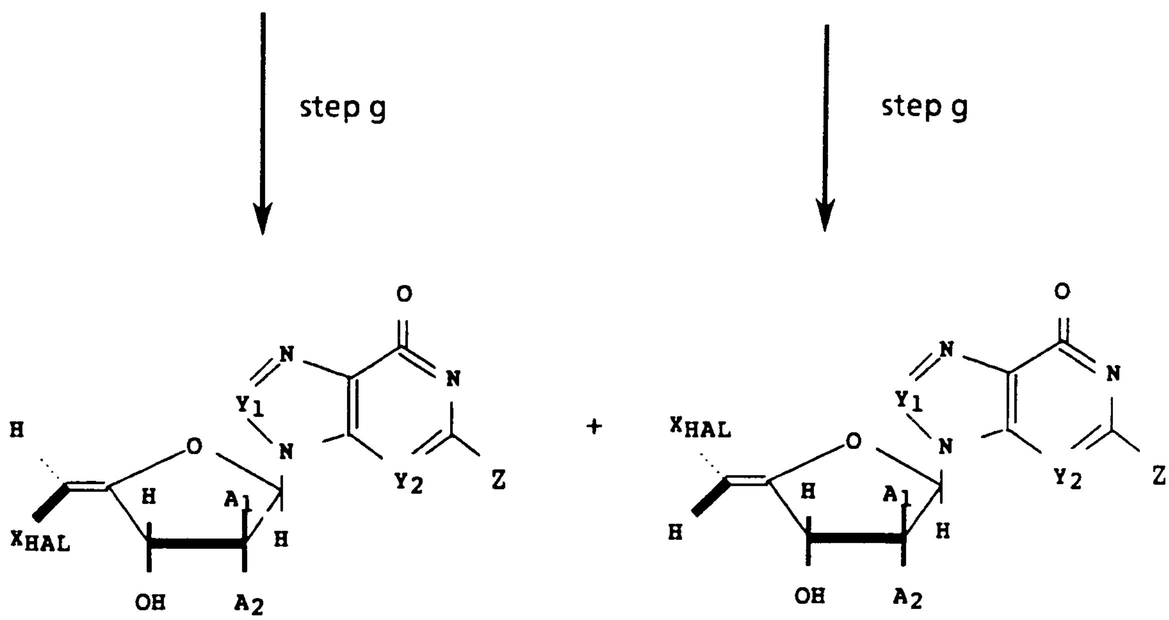


(12) (13)

In step a, reactive hydroxy groups of the appropriate inosine/guanosine derivative (14), other than the 5′-hydroxy, are blocked utilizing standard blocking agents well known in the art as described for Scheme B. Where $A_2$ is hydroxy, it is preferred to block the 2′- and 3′-hydroxy groups with a 2′,3′-O-isopropylidene blocking group. Where $A_2$ is not hydroxy, it is preferred to block the 3′-hydroxy and any 2′-hydroxy (wherein $A_1$ is hydroxy) with a benzoyl group. Where a 2′,3′-O-isopropylidene blocking group is not utilized, it is preferred to block the 3′-hydroxy and any 2′-hydroxy (wherein $A_1$ is hydroxy) after the reaction described in step b.

In step b, the 5′-hydroxy of the appropriately blocked 5′-hydroxy derivative (15) is subjected to a substitution reaction in which an alkyl-thio group replaces the 5′-hydroxy group to form the corresponding sulfide (16). The preferred sulfide is the 4-methoxyphenylsulfide which can be formed by reacting the appropriately blocked 5′-hydroxy derivative (15) with 4-methoxyphenyl disulfide in the presence of tributylphosphine.

In step c, any reactive amino groups, such as the 2-amino group of guanine or deoxyguanine, can be blocked as described for Scheme B. It is preferred to block the 2-amino group of guanine or deoxyguanine with an acetyl group. In addition, Where a 2′,3′-O-isopropylidene blocking group is not utilized in step a, the 3′-hydroxy and any 2′-hydroxy (wherein $A_1$ is hydroxy) can be blocked as described above.

In step d, the appropriately blocked sulfide (17) is oxidized to the corresponding sulfinyl derivative (18) utilizing standard oxidizing agents well known and appreciated in the art such as 3-chloroperbenzoic acid.

In step e, the 5′-carbon of the sulfinyl derivative (18) is halogenated using a halogenating agent, such as the fluorinating agent diethylaminosulfur trifluoride (DAST), or the chlorinating agent sulfuryl chloride in the presence of a base such as pyridine, to yield the corresponding 5′-halosulfinyl derivative (19). The preferred fluorinating agent is DAST and the preferred chlorinating agent is sulfuryl chloride. Where DAST is utilized

as the fluorinating agent, the fluorinated product must be re-oxidized after treatment with DAST with an equimolar amount of an oxidizing agent, such as 3-chloroperbenzoic acid, in order to provide the 5′-halo-sulfinyl derivative (19).

In step f, the sulfinyl group is then eliminated to yield the appropriately blocked 4′-vinylhalo derivatives (20 and 21) by heating the 5′-halo-sulfinyl derivative (19) in the presence of a base such as diisopropylethyl amine.

In step g, the blocking groups of the appropriately blocked 4′-vinylhalo derivatives (20 and 21) are removed according to conventional procedures and techniques well known and appreciated in the art such as those described for Scheme B. The (Z) and (E) isomers of the 4′-vinylhalo-inosine/guanosine derivative or the 4′-vinylhalo-deoxyinosine/guanosine derivative, i.e., (12) and (13), are thus formed. These isomers can be separated by conventional resolution techniques well known and appreciated in the art.

Starting materials for use in the general synthetic procedure outlined in Scheme C are readily available to one of ordinary skill in the art. For example, certain starting materials for various compounds of formula (1) wherein $A_2$ is H or OH, $Y_1$ is CH, $Y_2$ is N and Z is H are commercially available. Additional starting materials can be prepared by the use of conventional methods and analogous techniques which are well known and appreciated in the art.

The following example presents a typical synthesis as described by Scheme C. This example is understood to be illustrative only and is not intended to limit the scope of the present invention in any way.

EXAMPLE 4

(Z)- and (E)-4′,5′-Didehydro-5′-deoxy-5′-fluoroguanosine

Step a: 2′,3′-O-isopropylidene-guanosine

The title compound is commercially available.

Step b: 5′-Deoxy-2′,3′-O-isopropylidene-5′-[(4-methoxyphenyl)thio]guanosine

To a mixture of 2′,3′-O-isopropylideneguanosine (16.1 g, 0.05 moles) and 4-methoxyphenyl disulfide (26.0 g, 0.094 moles) in dry pyridine (125 mL), add tributylphosphine (23.3 mL, 0.094 moles) via syringe. Stir under nitrogen overnight. Add water and remove solvents *in vacuo* in a rotary evaporator. Stir the residue in a mixture of water and cyclohexane. Decant the cyclohexane layer. Repeat the cyclohexane extraction two more times. Extract the water layer with ethyl acetate. Dry the ethyl acetate solution with $MgSO_4$ and concentrate to 100 mL or less. Add chloroform to the ethyl acetate solution and cool. Collect crystals of the title compound.

Step c: $N^2$-acetyl-5′-Deoxy-2′,3′-O-isopropylidene-5′-[(4-methoxyphenyl)thio]guanosine

To 5′-Deoxy-2′,3′-O-isopropylidene-5′-[(4-methoxyphenyl)thio]guanosine (10 g, 0.022 moles) in 50 mL of pyridine, add 4.1 mL (2 equivalent) of acetic anhydride and stir overnight. Pour the mixture into water and extract with ethyl acetate. Dry the ethyl acetate extract with $MgSO_4$ and remove the solvents *in vacuo* to give the title compound.

Step d: $N^2$-acetyl-5′-Deoxy-2′,3′-O-isopropylidene-5′-[(4-methoxyphenyl)sulfoxyl]guanosine

To a solution of $N^2$-acetyl-5′-deoxy-2′,3′-O-isopropylidene-5′-[(4-methoxyphenyl)thio]guanosine (5 g, 0.01 moles) in 40 mL dichloromethane cooled in an ice bath, add dropwise a solution of 2.0 g (0.01 moles) of 85% 3-chloroperbenzoic acid in 40 mL dichloromethane. Stir the reaction mixture for 2 hours and partition the mixture between 400 mL chloroform and a saturated aqueous sodium bicarbonate solution. Dry the organic layer over $MgSO_4$ and evaporate the solution to dryness. Chromatograph the residue on 200 g silica gel eluting with ethyl acetate/methanol. Concentrate the eluant to yield the title compound.

Step e: $N^2$-acetyl-5′-Deoxy-5′-dehydro-2′,3′-O-isopropylidene-5′-fluoro-5′-[4-methoxyphenyl sulfoxyl]-guanosine

To 3.0 g of $N^2$-acetyl-5′-deoxy-2′,3′-O-isopropylidene-5′-[4-methoxyphenylsulfoxyl]guanosine (5.96 mmol) in 15 mL of 1,2-dichloroethane, add 3.15 mL (23.8 mmol) of diethylaminosulfur trifluoride. Stir the

reaction mixture at 45 ° C for 2 hours, cool and pour into saturated aqueous sodium bicarbonate. Extract the mixture with 200 mL chloroform. Dry the extract over $MgSO_4$ and evaporate the solvents *in vacuo*. Redissolve the residue in dichloromethane and evaporate the solvents *in vacuo*. Dissolve the residue in 25 mL of dichloromethane and cool in an ice bath. To this solution, add dropwise a solution of 0.97 g of 85% 3-chloroperbenzoic acid and stir for 2 hours. Partition the reaction mixture between 200 mL chloroform and saturated aqueous sodium bicarbonate. Dry the organic layer over $MgSO_4$ and evaporate the solvents to dryness. Chromatograph the residue on 100 g silica gel eluting sequentially with cyclohexane/ethyl acetate, ethyl acetate and ethyl acetate/methanol to provide the title compound.

Step f: (Z)- and (E)-$N^2$-acetyl-5′-Deoxy-4′,5′-didehydro-2′,3′-O-isopropylidene-5′-fluoroguanosine

Reflux a solution of 1.8 g of $N^2$-acetyl-5′-deoxy-5′-dehydro-2′,3′-O-isopropylidene-5′-fluoro-5′-[4-methoxyphenylsulfoxyl]guanosine in 35 mL diglyme and 2.5 g diisopropylethylamine for 24 hours. Remove solvents by distillation on a Kugelrohr apparatus at 1 mm Hg. Chromatograph the residue on 50 g silica gel eluting sequentially with cyclohexane/ethyl acetate, ethyl acetate and ethyl acetate/methanol to provide a mixture of the (Z) and (E) isomers of the title compound.

Step g: (Z)- and (E)-5′-Deoxy-4′,5′-didehydro-5′-fluoroguanosine

Place 0.5 g of (Z)- and (E)-$N^2$-acetyl-5′-deoxy-4′,5′-didehydro-2′,3′-O-isopropylidene-5′-fluoroguanosine (0.136 mmol) in 15 mL ethanol, cool to 0 ° C and saturate with ammonia in a pressure tube. Cap the tube and keep it at room temperature overnight. Evaporate the solvent and stir the residue in a few mL of ethyl acetate. Filter the mixture and dissolve the filter cake in 5 mL of trifluoroacetic acid/water (4/l, v/v). Stir the mixture at room temperature for 1 hour and evaporate *in vacuo*. Stir the residue in a few mL of ethyl acetate and filter to afford a 2:1 mixture of the (Z) and (E) isomers, respectively, of the title compound. Separate the isomers on a Bio-Rad® AG 1-1X resin (OH-form) under conditions described for guanosine by Dekker [*J.Am. Chem.Soc.* 87, 4027-29 (1965)].

The following specific compounds can be made by procedures analogous to those described in Example 8:

(Z) and (E)-4′,5′-Didehydro-5′-deoxy-5′-chloro-guanosine
(Z) and (E)-4′,5′-Didehydro-5′-deoxy-5′-chloro-inosine
(Z) and (E)-4′,5′-Didehydro-2′,5′-dideoxy-5′-chloro-guanosine
(Z) and (E)-4′,5′-Didehydro-2′,5′-dideoxy-5′-chloro-inosine
(Z) and (E)-4′,5′-Didehydro-5′-deoxy-5′-fluoro-inosine
(Z) and (E)-4′,5′-Didehydro-2′,5′-dideoxy-5′-fluoro-guanosine
(Z) and (E)-4′,5′-Didehydro-2′,5′-dideoxy-5′-fluoro-inosine.

The 4′-vinylhalo-inosine/guanosine derivatives of the formula (1) wherein both of $X_1$ and $X_2$ are halogen, can be prepared according to the procedure described in Scheme D. This procedure can be utilized to provide compounds of formula (1) wherein $X_1$ and $X_2$ are the same halogen, as well as to provide compounds of formula (1) wherein $X_1$ and $X_2$ are different halogens. This procedure is particularly useful to provide compounds of formula (1) wherein both $X_1$ and $X_2$ are chlorine atoms and wherein one of $X_1$ and $X_2$ is a fluorine atom and the other is a chlorine atom.

Scheme D

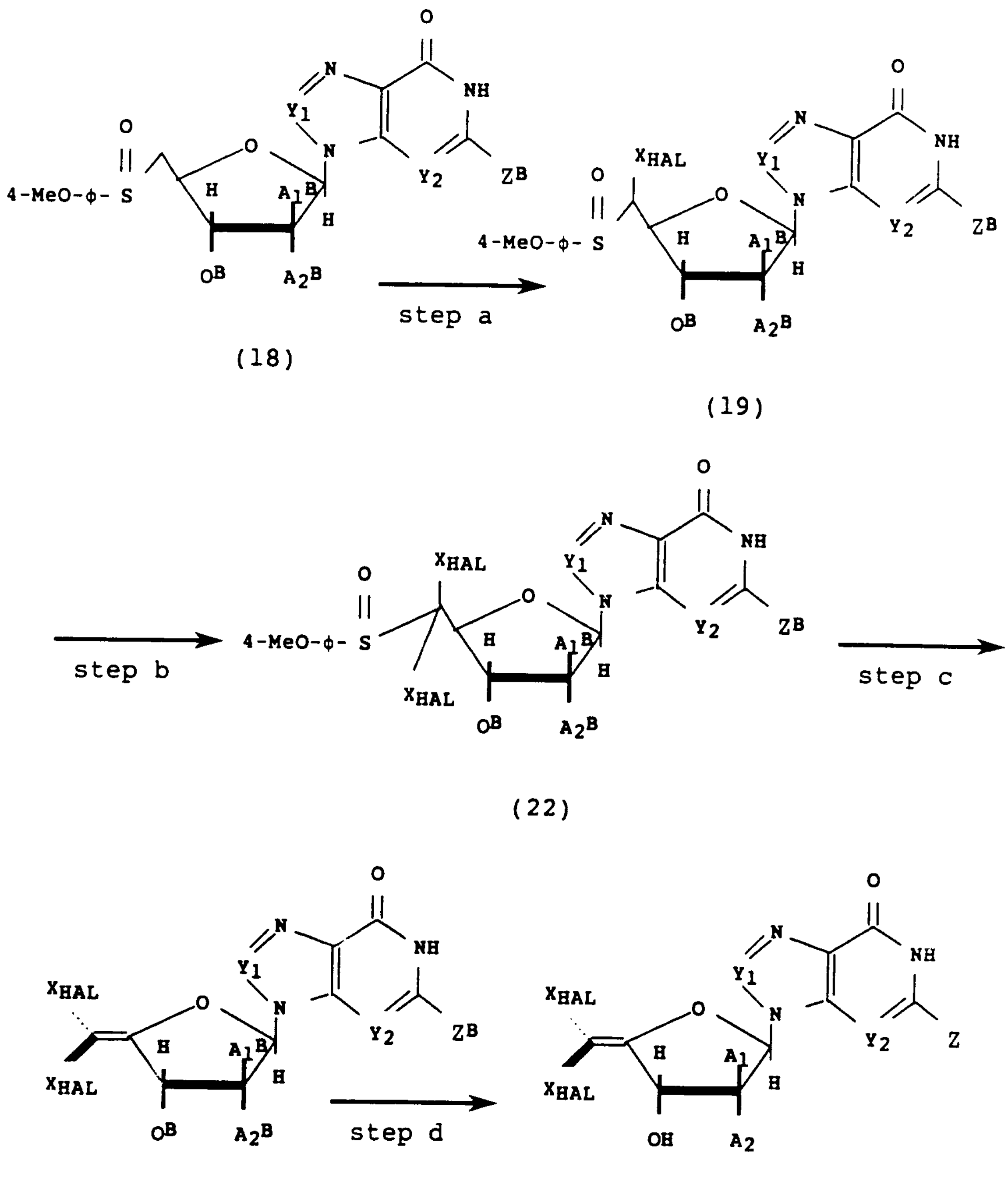


(18) (19) (22) (23) (24)

In step a, the 5'-carbon of the sulfinyl derivative (18), which is prepared as described in Scheme C, is halogenated using a halogenating agent to yield the corresponding 5'-halo-sulfinyl derivative (19). Where fluorination is desired, it is preferred to treat the sulfinyl derivative (18) with the fluorinating agent DAST followed by reoxidation with 3-chloroperbenzoic acid as described for Scheme C. Where chlorination is desires, it is preferred to treat the sulfinyl derivative (18) with the chlorinating agent sulfuryl chloride in the presence of a base such as pyridine.

In step b, halogenation of the 5'-carbon of the 5'-halo-sulfinyl derivative (19) is continued so as to provide a 5',5'-dihalo-sulfinyl derivative (22). Where compounds of formula (1) are desired wherein $X_1$ and

$X_2$ are both chlorine atoms, the sulfinyl derivative (18), depicted in step a, is treated with 2 equivalents of sulfuryl chloride to yield the corresponding 5′,5′-dichloro-sulfinyl derivative. Where compounds of formula (1) are desired wherein $X_1$ and $X_2$ are different halogens, the sulfinyl derivative (18) is treated with 1 equivalent of the appropriate halogenating agent. The 5′-halo-sulfinyl derivative (19) is isolated and then treated with 1 equivalent of the appropriate different halogenating agent.

For example, where compounds of formula (1) are desired wherein one of $X_1$ and $X_2$ is a fluorine atom and the other is a chlorine atom, the sulfinyl derivative (18) is treated with 1 equivalent of a fluorinating agent such as DAST, and reoxidized as described above, to yield the corresponding 5′-fluoro-sulfinyl derivative. The 5′-fluoro-sulfinyl derivative is then chlorinated with 1 equivalent of a chlorinating agent such as sulfuryl chloride to yield the corresponding 5′-fluoro-5′-chloro-sulfinyl derivative. For example, the 5′-fluoro-sulfinyl derivative (19) can be reacted with sulfuryl chloride in dichloromethane in the presence of pyridine.

In step c, the sulfinyl group of the 5′,5′-dihalosulfinyl derivative (22) is eliminated as described in Scheme C, step f, to yield the appropriately blocked 4′-vinyl-dihalo derivative (23).

In step d, the blocking groups of the appropriately blocked 4′-vinyl-dihalo derivative (23) are removed as described in Scheme C, step g, to yield the 4′-vinyl-dihalo derivative (24).

It is of course understood that where $X_1$ and $X_2$ are each different halogens, the 4′-vinyl-dihalo derivative (24) will exist as a mixture of the (Z) and (E) isomers. These isomers can be readily separated by conventional techniques and procedures as are well known and appreciated in the art.

The following example presents a typical synthesis as described by Scheme C. This example is understood to be illustrative only and is not only intended to limit the scope of the present invention in any way.

EXAMPLE 5

(Z)- and (E)-4′,5′-Didehydro-5′-deoxy-5′-fluoro-5′-chloroguanosine

Step a: $N^2$-acetyl-5′-Deoxy-2′,3′-O-isopropylidene-5′-fluoro-5′-[4-methoxyphenylsulfoxy]guanosine

To 3.0 g of $N^2$-acetyl-5′-deoxy-2′,3′-O-isopropylidene-5′-[4-methoxyphenylsulfoxyl]guanosine (5.96 mmol) in 15 mL of 1,2-dichloroethane, add 3.15 mL (23.8 mmol) of diethylaminosulfur trifluoride. Stir the reaction mixture at 45 °C for 2 hours, cool and pour into saturated aqueous sodium bicarbonate. Extract the mixture with 200 mL chloroform. Dry the extract over $MgSO_4$ and evaporate the solvents *in vacuo*. Redissolve the residue in dichloromethane and evaporate the solvents *in vacuo*. Dissolve the residue in 25 mL of dichloromethane and cool in an ice bath. To this solution, add dropwise a solution of 0.97 g of 85% 3-chloroperbenzoic acid and stir for 2 hours. Partition the reaction mixture between 200 mL chloroform and saturated aqueous sodium bicarbonate. Dry the organic layer over $MgSO_4$ and evaporate the solvents to dryness. Chromatograph the residue on 100 g silica gel eluting sequentially with cyclohexane/ethyl acetate, ethyl acetate and ethyl acetate/methanol to provide the title compound.

Step b: $N^2$-acetyl-5′-Deoxy-2′,3′-O-isopropylidene-5′-fluoro-5′-chloro-5′-[4-methoxyphenylsulfoxyl]guanosine

To $N^2$-acetyl-5′-deoxy-2′,3′-O-isopropylidene-5′-fluoro-5′-[4-methoxyphenylsulfoxyl]guanosine (0.89 g, 1.7 mmol) in 6.5 mL of dry dichloromethane add 0.32 mL (4.0 mmol) of pyridine and cool the mixture in an ice bath under nitrogen. To this mixture, add 0.18 mL (1.9 mmol) of $SO_2Cl_2$ (sulfuryl chloride) and stir the mixture for 20 minutes. Remove the solvents *in vacuo* to afford a foam. Percolate the product through a silica gel column eluting with dichloromethane followed by ethyl acetate/dichloromethane (1:4, v/v). Repeat the percolation procedure to yield the title compound as a foam. Triturate the product with dichloromethane/cyclohexane to yield a solid.

Step c: (Z)- and (E)-$N^2$-acetyl-5′-Deoxy-4′,5′-didehydro-2′,3′-O-isopropylidene-5′-fluoro-5′-chloro-guanosine

Reflux a solution of 1.0 g of $N^2$-acetyl-5′-deoxy-2′,3′-O-isopropylidene-5′-fluoro-5′-chloro-5′-[4-methoxyphenylsulfoxy]guanosine in 35 mL diglyme and 2.5 g diisopropylethylamine for 24 hours. Remove solvents by distillation on a Kugelrohr apparatus at 1 mm Hg. Chromatograph the residue on 50 g silica gel eluting sequentially with cyclohexane/ethyl acetate, ethyl acetate and ethyl acetate/methanol to provide a mixture of the (Z) and (E) isomers of the title compound.

Step d: (Z)- and (E)-5′-Deoxy-4′,5′-didehydro-5′-fluoro-5′-chloro-guanosine

Place 0.4 g of (Z)- and (E)-$N^2$-acetyl-5′-deoxy-4′,5′-didehydro-2′,3′-O-isopropylidene-5′-fluoro-5′-chloro-guanosine (1.0 mmol) in 15 mL ethanol, cool to 0 ° C and saturate with ammonia in a pressure tube. Cap the tube and keep it at room temperature overnight. Evaporate the solvent and stir the residue in a few mL of ethyl acetate. Filter the mixture and dissolve the filter cake in 5 mL of trifluoroacetic acid/water (4/1, v/v). Stir the mixture at room temperature for 1 hour and evaporate *in vacuo*. Stir the residue in a few mL of ethyl acetate and filter to afford a mixture of the (Z) and (E) isomers of the title compound. Separate the isomers on a Bio-Rad® AG 1-1X resin (OH-form) under conditions described for guanosine by Dekker [*J.Am. Chem.Soc.* 87, 4027-29 (1965)].

The following specific compounds can be made by procedures analogous to those described in Example 8:

(Z)- and (E)-5′-Deoxy-4′,5′-didehydro-5′-fluoro-5′-chloroinosine
(Z)- and (E)-2′,5′-Dideoxy-4′,5′-didehydro-5′-fluoro-5′-chloro-inosine
(Z)- and (E)-2′,5′-Dideoxy-4′,5′-didehydro-5′-fluoro-5′-chloro-guanosine
(Z)- and (E)-5′-Deoxy-4′,5′-didehydro-5′,5′-dichloro-inosine
(Z)- and (E)-2′,5′-Dideoxy-4′,5′-didehydro-5′,5′-dichlor-inosine
(Z)- and (E)-5′-Deoxy-4′,5′-didehydro-5′,5′-dichloro-guanosine
(Z)- and (E)-2′,5′-Dideoxy-4′,5′-didehydro-5′,5′-dichloro-guanosine.

The present invention further provides a method of treating a patient suffering from a protozoal infection comprising administering to said patient a therapeutically effective antiprotozoal amount of a compound of formula (1).

According to the present invention, a patient suffering from a protozoal infection is treated by providing an antiprotozoal effect. Treatment of a patient suffering from a protozoal infection is effected by administering to the patient a therapeutically effective antiprotozoal amount of a compound of formula (1). It is believed that this antiprotozoal effect is generated through an inhibition of purine nucleoside hydrolase, however it is understood that the present invention is not limited by any proposed mechanism by which it may operate.

The term "antiprotozoal effect" refers to an effect of controlling the growth or proliferation of the protozoa or in prolonging the survivability of the patient beyond that expected in the absence of such treatment. The growth or proliferation of a protozoa is controlled by slowing, interrupting, arresting or stopping its growth or proliferation. The term "controlling the growth or proliferation of the protozoa" therefore does not necessarily indicate a total elimination of the protozoa.

As used herein, the term "patient" refers to a warm-blooded animal, such as a mammal, which is suffering from a protozoal infection. It is understood that humans are included within the scope of the meaning of the term.

A number of protozoa are parasitic and cause protozoal infections in warm-blooded animals such as humans. These parasitic protozoa can live either intracellularly or extracellularly in the patient or host animal and are the cause of a variety of recognized diseases. For example, protozoa classified in the following genera can be parasitic and can infect warm-blooded animals:

Plasmodium (including such species as vivax, malariae, ovale, falciparum, knowlesi, berghei, vinckei, chabaudi, gallinaceum and lophurae);

Leishmania (including such species as donovani, tropica, braziliensis and mexicana);

Babesia (including such species as bovis, rodhaini and microti);

Trypanosoma (including those species of the class stercoraria such as cruzi and those species of the class salivaria such as rhodesiense, gambiense, brucei, evansi, equinum, equiperdum, congolense and vivax) Toxoplasma (including such species as gondii);

Theileria (including such species as parva);

Trichomonas (including such species as faginalis, foetus and gallinae);

Entamoeba (including such species as histolytica and invadens);

Pneumocystis (including such species as carinii);

Eimeria (including such species as tenella, necratrix and brunetti);

Giardia (including such species as lamblia); and Cryptosporidia.

All of the foregoing protozoa are known to infect warm-blooded animals and the particular diseases for which these protozoa are responsible are well known and are readily identifiable by those skilled in the art of diagnosis. Of particular importance in the end-use application of the compounds of formula (1) is the treatment of patients suffering from malaria. The disease malaria can be caused by infection of a human host by a variety of protozoa of the genus Plasmodium and of species such as vivax, malariae, ovale and

falciparum. Malaria in non-human hosts can be caused by infection by a variety of protozoa of the genus Plasmodium and of species such as knowlesi, berghei, vinckei, chabaudi, gallinaceum and lophurae.

In addition, other particularly important diseases which are caused by protozoal infections in humans, include Trypanosomiasis (which is caused by infections of the human host with protozoa of the genus Trypanosoma), Chagas' Disease (which is caused by infections of the human host with Trypanosoma cruzi), Leishmaniasis (which is caused by infections of the human host with protozoa of the genus Leishmania), Amebiasis (which is caused by infections of the human host with protozoa of the genus Entamoeba), Toxoplasmosis (which is caused by infections of the human host with protozoa of the genus Toxoplasma), as well as the opportunistic infection of human hosts by Pneumocystis carinii.

In effecting treatment of a patient afflicted with a disease state described above, a compound of formula (1) can be administered in any form or mode which makes the compound bioavailable in therapeutically effective antiprotozoal amounts, including oral and parenteral routes. For example, compounds of formula (1) can be administered orally, topically, subcutaneously, intramuscularly, intravenously, transdermally, intranasally, rectally, and the like. Oral administration is generally preferred. One skilled in the art can readily select the proper form and mode of administration depending upon the particular characteristics of the compound selected the disease state to be treated, the stage of the disease, and other relevant circumstances.

As used herein, the term "therapeutically effective antiprotozoal amount" refers to an amount of the compound of formula (1) which is effective in providing an antiprotozoal effect. A therapeutically effective antiprotozoal amount can be readily determined by the attending diagnostician, as one skilled in the art, by the use of known techniques and by observing results obtained under analogous circumstances. In determining the therapeutically effective antiprotozoal amount, a number of factors are considered by the attending diagnostician, including, but not limited to: the species of mammal; its size, age, and general health; the specific disease involved; the degree of or involvement or the severity of the disease; the response of the individual patient; the particular compound administered; the mode of administration; the bioavailability characteristics of the preparation administered; the dose regimen selected; the use of concomitant medication; and other relevant circumstances.

A therapeutically effective antiprotozoal amount of a compound of formula (1) is expected to vary from about 0.1 milligram per kilogram of body weight per day (mg/kg/day) to about 100 mg/kg/day. Preferred amounts are expected to vary from about 1 to about 20 mg/kg/day. These ranges are particularly reflective of effective amounts upon oral administration but also are reflective of the operable ranges for parenteral administration.

The compounds can be administered alone or in the form of a pharmaceutical composition in combination with pharmaceutically acceptable carriers or excipients, the proportion and nature of which are determined by the solubility and chemical properties of the compound selected, the chosen route of administration, and standard pharmaceutical practice. The compounds of the invention, while effective themselves, may be formulated and administered in the form of their pharmaceutically acceptable acid addition salts for purposes of stability, convenience of crystallization, increased solubility and the like.

In another embodiment, the present invention provides compositions comprising an assayable amount of a compound of formula (1) in admixture with one or more inert carriers. These compositions are useful, for example, as assay standards, as convenient means of making bulk shipments, or as pharmaceutical compositions. An assayable amount of a compound of formula (1) is an amount which is readily measurable by standard assay procedures and techniques as are well known and appreciated by those skilled in the art. Assayable amounts of a compound of formula (1) will generally vary from about 0.001% to about 75% of the composition by weight. Inert carriers can be any material which does not degrade or otherwise covalently react with a compound of formula (1). Examples of suitable inert carriers are water; aqueous buffers, such as those which are generally useful in High Performance Liquid Chromatography (HPLC) analysis; organic solvents, such as acetonitrile, ethyl acetate, hexane and the like; and pharmaceutically acceptable carriers or excipients. These compositions are prepared by mixing the compound of formula (1) with the inert carriers utilizing techniques and methods which are well known and appreciated in the art.

More particularly, the present invention provides pharmaceutical compositions comprising a therapeutically effective antiprotozoal amount of a compound of formula (1) in admixture or otherwise in association with one or more pharmaceutically acceptable carriers or excipients.

The pharmaceutical compositions are prepared in a manner well known in the pharmaceutical art. The carrier or excipient may be a solid, semi-solid, or liquid material which can serve as a vehicle or medium for the active ingredient. Suitable carriers or excipients are well known in the art. The pharmaceutical composition may be adapted for oral or parenteral use and may be administered to the patient in the form of tablets, capsules, suppositories, solution, suspensions, or the like.

The compounds of the present invention may be administered orally, for example, with an inert diluent or with an edible carrier. They may be enclosed in gelatin capsules or compressed into tablets. For the purpose of oral therapeutic administration, the compounds may be incorporated with excipients and used in the form of tablets, troches, capsules, elixirs, suspensions, syrups, wafers, chewing gums and the like. These preparations should contain at least 4% of the compound of the invention, the active ingredient, but may be varied depending upon the particular form and may conveniently be between 4% to about 70% of the weight of the unit. The amount of the compound present in compositions is such that a suitable dosage will be obtained. Preferred compositions and preparations according to the present invention are prepared so that an oral dosage unit form contains between 5.0-300 milligrams of a compound of the invention.

The tablets, pills, capsules, troches and the like may also contain one or more of the following adjuvants: binders such as microcrystalline cellulose, gum tragacanth or gelatin; excipients such as starch or lactose, disintegrating agents such as alginic acid, Primogel, corn starch and the like; lubricants such as magnesium stearate or Sterotex; glidants such as colloidal silicon dioxide; and sweetening agents such as sucrose or saccharin may be added or a flavoring agent such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the present compounds, sucrose as a sweetening agent and certain preservatives, dyes and colorings and flavors. Materials used in preparing these various compositions should be pharmaceutically pure and non-toxic in the amounts used.

For the purpose of parenteral therapeutic administration, the compounds of the present invention may be incorporated into a solution or suspension. These preparations should contain at least 0.1% of a compound of the invention, but may be varied to be between 0.1 and about 50% of the weight thereof. The amount of the inventive compound present in such compositions is such that a suitable dosage will be obtained. Preferred compositions and preparations according to the present invention are prepared so that a parenteral dosage unit contains between 5.0 to 100 milligrams of the compound of the invention.

The solutions or suspensions may also include the one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

In addition, the present invention also provides a method of inhibiting a purine nucleoside hydrolase in a protozoa comprising contacting said protozoa with an effective purine nucleoside hydrolase inhibitory amount of a compound of formula (1). By contacting a protozoa with an effective purine nucleoside hydrolase inhibitory amount of a compound of formula (1), the purine nucleoside hydrolase of the protozoa is inhibited, thereby providing an antiprotozoal effect. Protozoa are contacted with a compound of formula (1), by causing the compound to be present in the medium surrounding the protozoa. Where a protozoa infects a host animal, the compound is caused to be present in the surrounding medium, either intracellularly or extracellularly, by administration to the infected animal in a manner as described above. The term "effective purine nucleoside hydrolase inhibitory amount" refers to that amount of a compound of formula (1) which is effective in substantially inhibiting the purine nucleoside hydrolase in the protozoa so as to provide an antiprotozoal effect. The concentration of the compound of formula (1) in the surrounding medium required to provide an effective purine nucleoside hydrolase inhibitory amount will generally vary from about 10 $\eta$M (nanomolar) to about 10 $\mu$M (micromolar) and will preferably be from about 10 $\eta$M to about 0.1 $\mu$M.

Protozoa can be contacted with a compound of the formula (1) in a variety of manners in order to produce the desired antiprotozoal effect. For example, a solution of the compound of the formula (1) can be placed directly on and surrounding the protozoa. A preferred manner of contact however is to administer the compound of formula (1) to a host suffering from a protozoal infection so that the compound is carried through the systemic circulation of the host and is thus placed in contact with the protozoa inside the host. The term "host" refers to any warm-blooded animal, including man, which is suffering from a protozoal infection in its blood or other tissues. The compound of formula (1) can be administered to the host in any manner which makes the compound bioavailable in effective amounts including for example, by topical, oral or parenteral administration.

As with any group of structurally related compounds which possesses a particular generic utility, certain groups and configurations are preferred for compounds of formula (1) in their end-use application..

With respect to the substituents $X_1$ and $X_2$, compounds of formula (1) wherein $X_1$ is fluoro and $X_2$ is hydrogen, and those wherein $X_1$ is hydrogen and $X_2$ is fluoro, are generally preferred. With respect to the substituent $A_1$, compounds of formula (1) wherein $A_1$ is hydrogen are generally preferred. With respect to the substituent $A_2$, compounds of formula (1) wherein $A_2$ is hydroxy are generally preferred. Furthermore, compounds of formula (1) wherein $Y_1$ is a CH group and $Y_2$ is nitrogen are generally preferred.

## Claims

### Claims for the following Contracting States : AT, BE, CH, DE, DK, FR, GB, IT, LI, LU, NL, SE

**1.** A compound of the formula 1

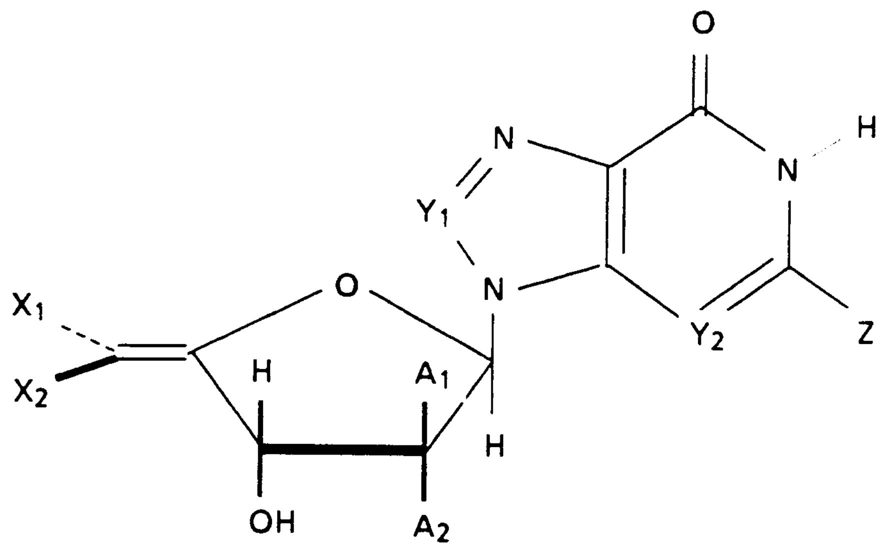

(1)

wherein

| | |
|---|---|
| $X_1$ and $X_2$ | are each independently hydrogen or halogen with the proviso that at least one of $X_1$ and $X_2$ is a halogen atom, |
| $A_1$ and $A_2$ | are each independently hydrogen, halogen or hydroxy with the provisos that where $A_1$ is hydroxy, $A_2$ is hydrogen, and where $A_2$ is hydroxy, $A_1$ is hydrogen, |
| $Y_1$ | is nitrogen, a CH group, a CCl group, a CBr group or a $CNH_2$ group, |
| $Y_2$ | is nitrogen or a CH group, and |
| Z | is hydrogen, halogen, or $NH_2$. |

**2.** A compound of claim 1 wherein $Y_1$ is a CH group.

**3.** A compound of claim 2 wherein $Y_2$ is an NH group.

**4.** A compound of claim 3 wherein $Y_2$ is nitrogen.

**5.** A compound of claim 4 wherein $A_1$ is hydrogen and $A_2$ is hydroxy.

**6.** A compound of claim 5 wherein $X_1$ is halogen and $X_2$ is hydrogen.

**7.** A compound of claim 5 wherein $X_1$ is hydrogen and $X_2$ is halogen.

**8.** A compound of claim 6 wherein $X_1$ is chlorine.

**9.** A compound of claim 7 wherein $X_2$ is chlorine.

**10.** A compound of claim 8 or 9 wherein Z is hydrogen.

**11.** A compound of claim 8 or 9 wherein Z is $NH_2$.

**12.** A process for preparing a compound of the formula 3

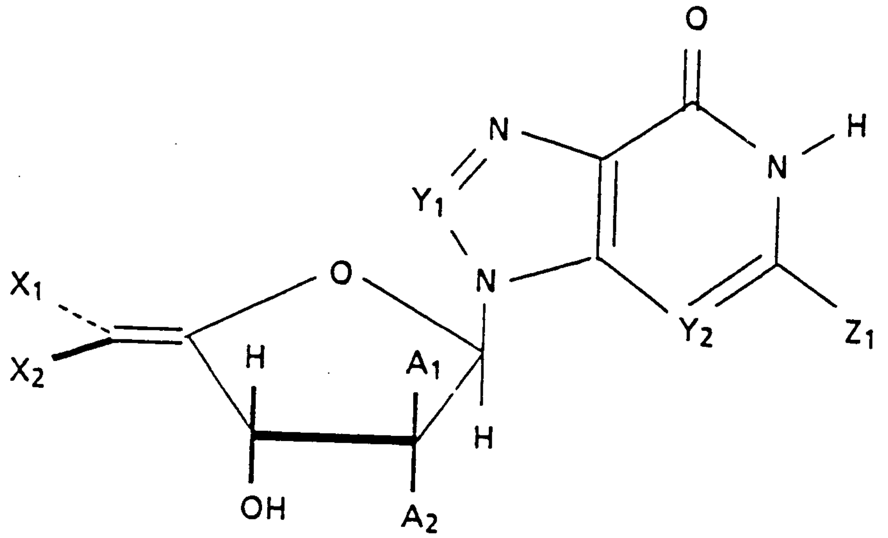

(3)

wherein $X_1$, $X_2$, $A_1$, $A_2$, $Y_1$ and $Y_2$ have the same meaning as in claim 1, and $Z_1$ is hydrogen or halogen, comprising reacting an acidic oxidative agent with a compound of the formula 2

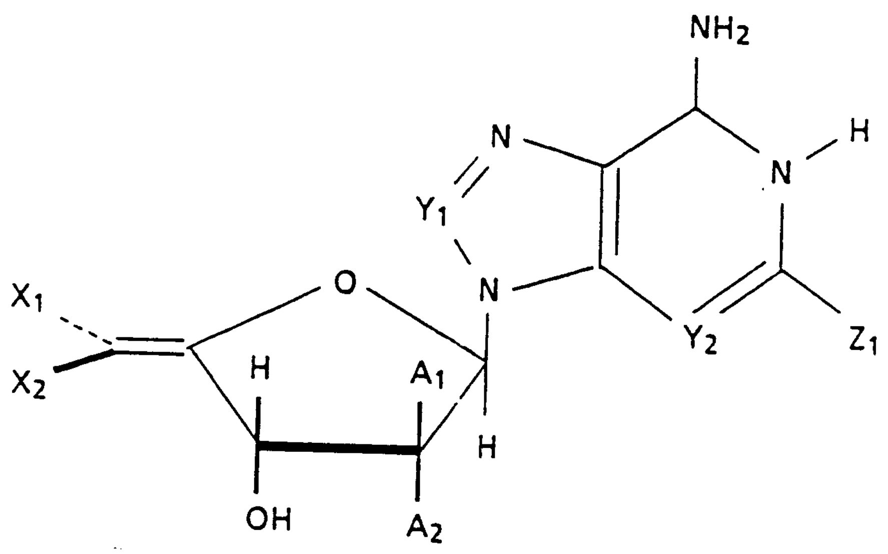

(2)

wherein the substituents $X_1$, $X_2$, $A_1$, $A_2$, $Y_1$, $Y_2$ and $Z_1$ have the above meaning.

**13.** A process for preparing a compound of the formula 1

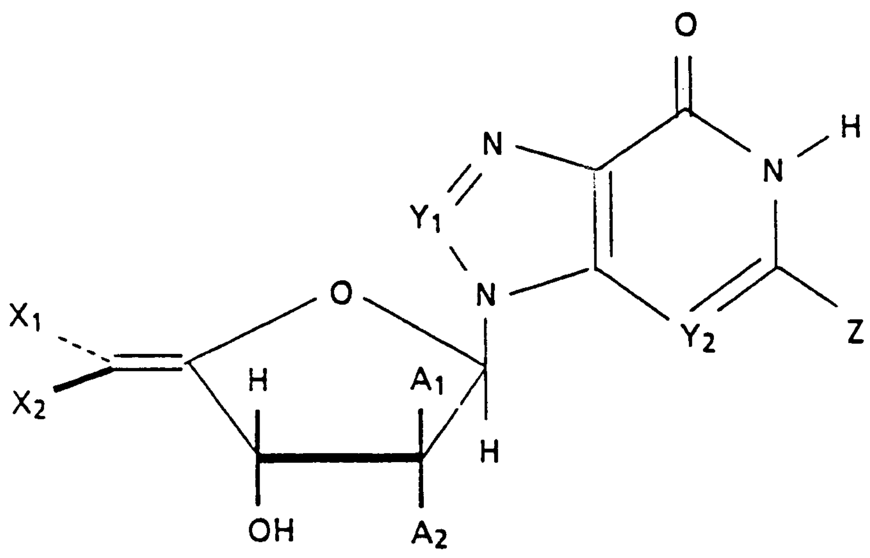

(1)

wherein $X_1$, $X_2$, $A_1$, $A_2$, $Y_1$, $Y_2$ and Z have the same meaning as in claim 1, comprising reacting a compound of the formula

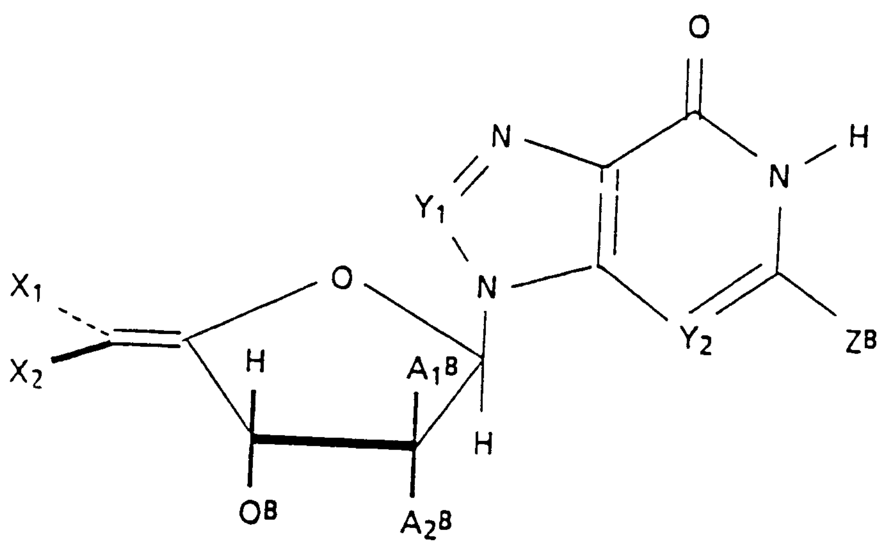

wherein $X_1$, $X_2$, $Y_1$, and $Y_2$ have the above meaning $O^B$, $A_1{}^B$ and $A_2{}^B$ represent OH, $A_1$ and $A_2$ groups, respectively, bearing hydroxy-blocking groups where appropriate, and $Z^B$ represents a Z group bearing an amino-blocking group where appropriate,

(a) with an acid to remove any hydroxy-protecting groups and

(b) with a base to remove any amino-protecting groups.

**14.** A method of inhibiting a purine nucleoside hydrolase in a protozoa comprising contacting said protozoa in vitro with an effective purine nucleoside hydrolase inhibitory amount of a compound of claim 1.

**15.** Use of a compound of any of claims 1 to 11 for the preparation of a pharmaceutical composition.

**16.** Use of a compound of any of claims 1 to 11 for the preparation of a pharmaceutical composition for treating a patient suffering from a protozoal infection.

**17.** Use according to claim 16, wherein the protozoal infection is by a protozoa of the genera Plasmodium, Trypanosoma, Leishmania, Trichomonas, Babesia, Toxoplasma, Pneumocystis or Theileria.

**18.** Use according to claim 16, wherein the patient is suffering from Ambiasis, Malaria, Leishmaniasis, Trypanosomiasis, Toxoplasmosis or a Pneumocysis carinii infection.

**19.** Use according to claim 16, wherein the patient is suffering from Chagas' Disease.

**20.** Use according to claim 16, wherein the patient is suffering from malaria.

**21.** A composition comprising an assayable amount of a compound of any of claims 1 to 11 in admixture with one or more inert carriers.

**22.** A pharmaceutical composition comprising a therapeutically effective amount of a compound of any of claims 1 to 11 in admixture with one or more pharmaceutically acceptable carriers or excipients.

**23.** A pharmaceutical composition according to claim 22 for treating a patient suffering from a protozoal infection.

**Claims for the following Contracting State : ES**

**1.** A process for preparing a compound of the formula 1

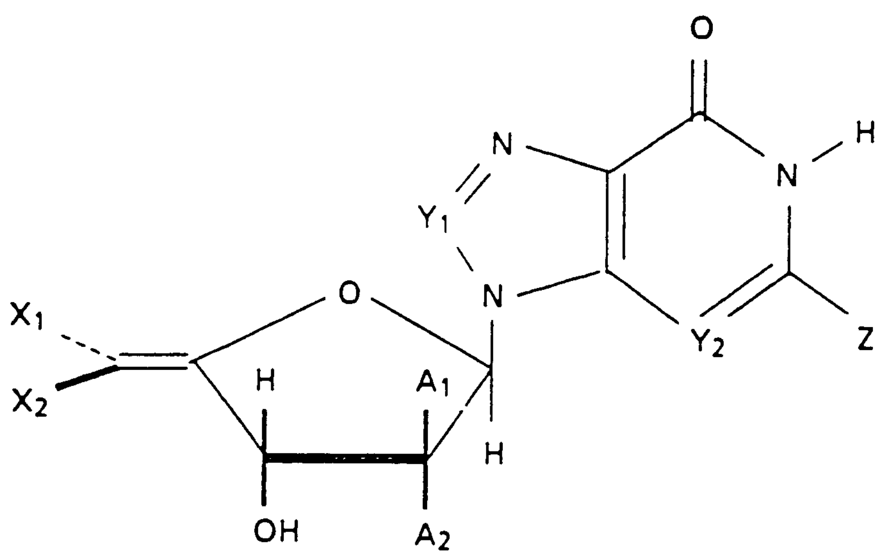

(1)

wherein

| | |
|---|---|
| $X_1$ and $X_2$ | are each independently hydrogen or halogen with the proviso that at least one of $X_1$ and $X_2$ is a halogen atom, |
| $A_1$ and $A_2$ | are each independently hydrogen, halogen or hydroxy with the provisos that where $A_1$ is hydroxy, $A_2$ is hydrogen, and where $A_2$ is hydroxy, $A_1$ is hydrogen, |
| $Y_1$ | is nitrogen, a CH group, a CCl group, a CBr group or a $CNH_2$ group, |
| $Y_2$ | is nitrogen or a CH group, and |
| Z | is hydrogen, halogen, or $NH_2$ |

comprising

1.1 when preparing compound 1 wherein Z is other than $NH_2$ reacting an oxidative agent with a compound of the formula 2

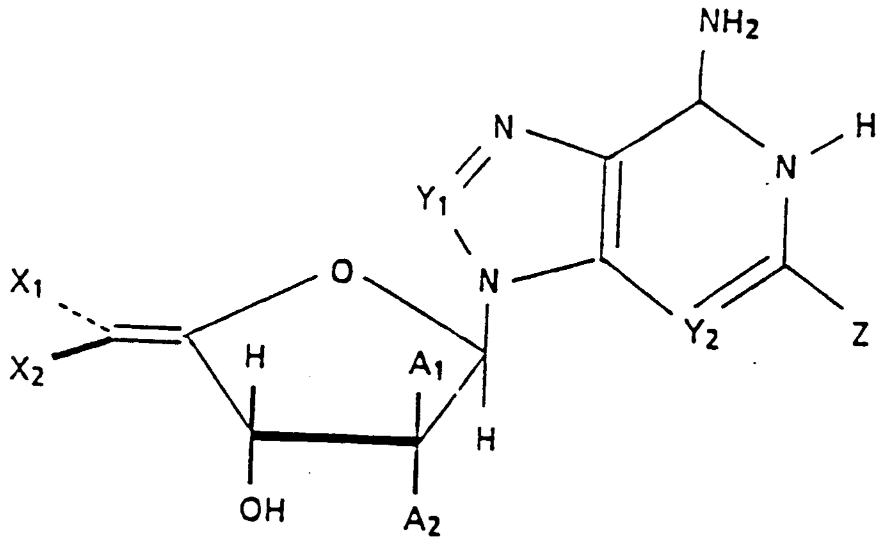

(2)

wherein the substituents $X_1$, $X_2$, $A_1$, $A_2$, $Y_1$, $Y_2$ and Z have the above meaning; and
1.2 when preparing compound 1 wherein Z is hydrogen, halogen or $NH_2$ reacting a compound of the formula

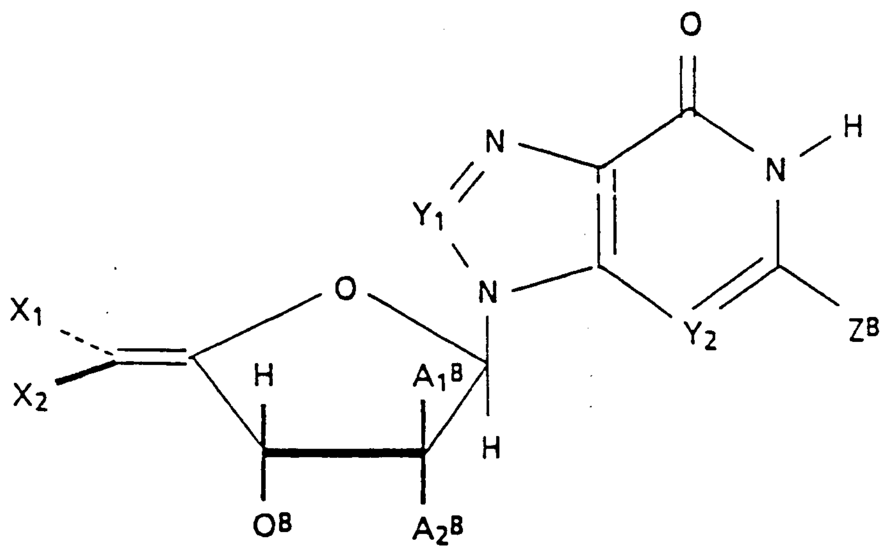

wherein $O^B$, $A_1{}^B$ and $A_2{}^B$ represent OH, $A_1$ and $A_2$ groups, respectively, bearing hydroxy-blocking groups where appropriate, and $Z^B$ represents a Z group bearing an amino-blocking group where appropriate,
(a) with an acid to remove any hydroxy-protecting groups and
(b) with a base to remove any amino-protecting group.

**2.** A process of claim 1 wherein $Y_1$ is a CH group.

**3.** A process of claim 2 wherein $Y_2$ is an NH group.

**4.** A process of claim 3 wherein $Y_2$ is nitrogen.

**5.** A process of claim 4 wherein $A_1$ is hydrogen and $A_2$ is hydroxy.

6. A process of claim 5 wherein $X_1$ is halogen and $X_2$ is hydrogen.

7. A process of claim 5 wherein $X_1$ is hydrogen and $X_2$ is halogen.

8. A process of claim 6 wherein $X_1$ is chlorine.

9. A process of claim 7 wherein $X_2$ is chlorine.

10. A process of claim 8 or 9 wherein Z is hydrogen.

11. A process of claim 8 or 9 wherein Z is $NH_2$.

12. A method of inhibiting a purine nucleoside hydrolase in a protozoa comprising contacting said protozoa in vitro with an effective purine nucleoside hydrolase inhibitory amount of a compound as prepared according to claim 1.

13. A composition comprising an assayable amount of a compound prepared according to any of claims 1 to 11 in admixture with one or more inert carriers.

**Claims for the following Contracting State : GR**

1. A compound of the formula 1

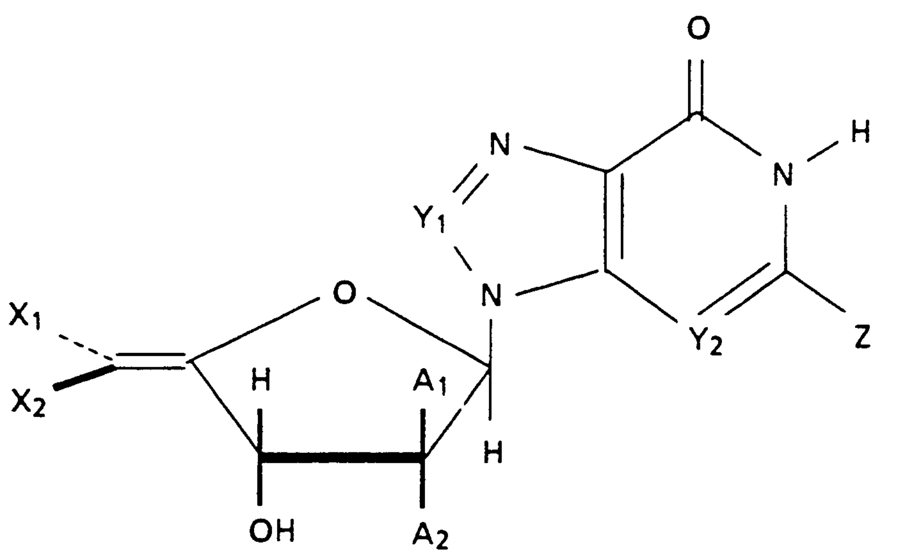

(1)

wherein

| | |
|---|---|
| $X_1$ and $X_2$ | are each independently hydrogen or halogen with the proviso that at least one of $X_1$ and $X_2$ is a halogen atom, |
| $A_1$ and $A_2$ | are each independently hydrogen, halogen or hydroxy with the provisos that where $A_1$ is hydroxy, $A_2$ is hydrogen, and where $A_2$ is hydroxy, $A_1$ is hydrogen, |
| $Y_1$ | is nitrogen, a CH group, a CCl group, a CBr group or a $CNH_2$ group, |
| $Y_2$ | is nitrogen or a CH group, and |
| Z | is hydrogen, halogen, or $NH_2$. |

2. A compound of claim 1 wherein $Y_1$ is a CH group.

3. A compound of claim 2 wherein $Y_2$ is an NH group.

4. A compound of claim 3 wherein $Y_2$ is nitrogen.

5. A compound of claim 4 wherein $A_1$ is hydrogen and $A_2$ is hydroxy.

6. A compound of claim 5 wherein $X_1$ is halogen and $X_2$ is hydrogen.

7. A compound of claim 5 wherein $X_1$ is hydrogen and $X_2$ is halogen.

8. A compound of claim 6 wherein $X_1$ is chlorine.

9. A compound of claim 7 wherein $X_2$ is chlorine.

10. A compound of claim 8 or 9 wherein Z is hydrogen.

11. A compound of claim 8 or 9 wherein Z is $NH_2$.

12. A process for preparing a compound of the formula 3

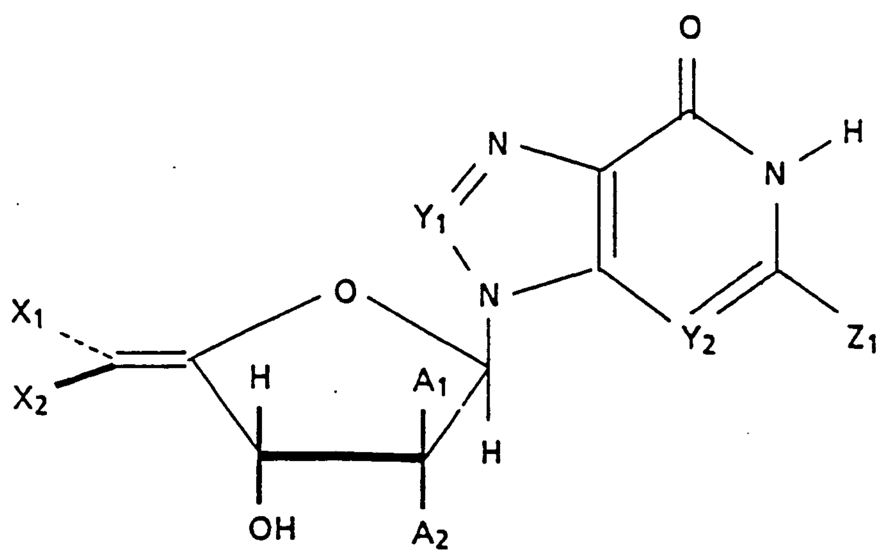

(3)

wherein $X_1$, $X_2$, $A_1$, $A_2$, $Y_1$ and $Y_2$ have the same meaning as in claim 1, and
$Z_1$ is hydrogen or halogen,
comprising reacting an acidic oxidative agent with a compound of the formula 2

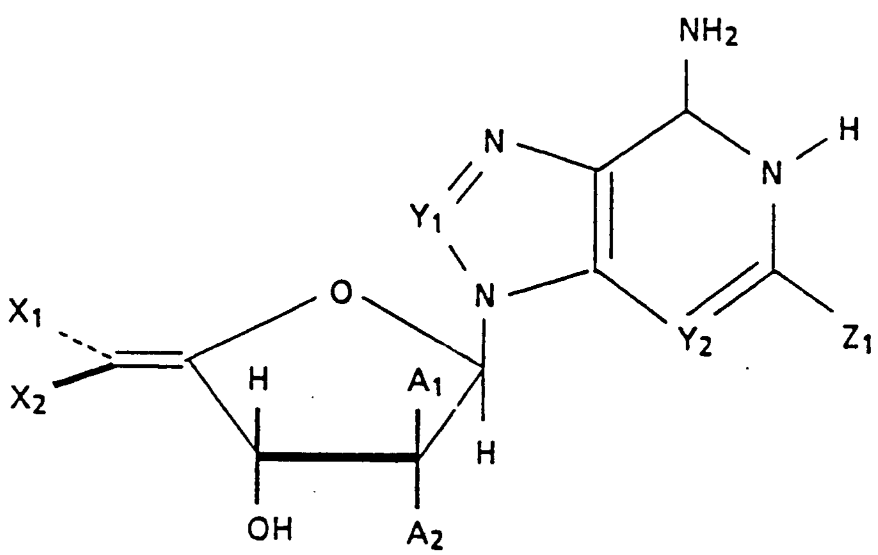

(2)

wherein the substituents $X_1$, $X_2$, $A_1$, $A_2$, $Y_1$, $Y_2$ and $Z_1$ have the above meaning.

**13.** A process for preparing a compound of the formula 1

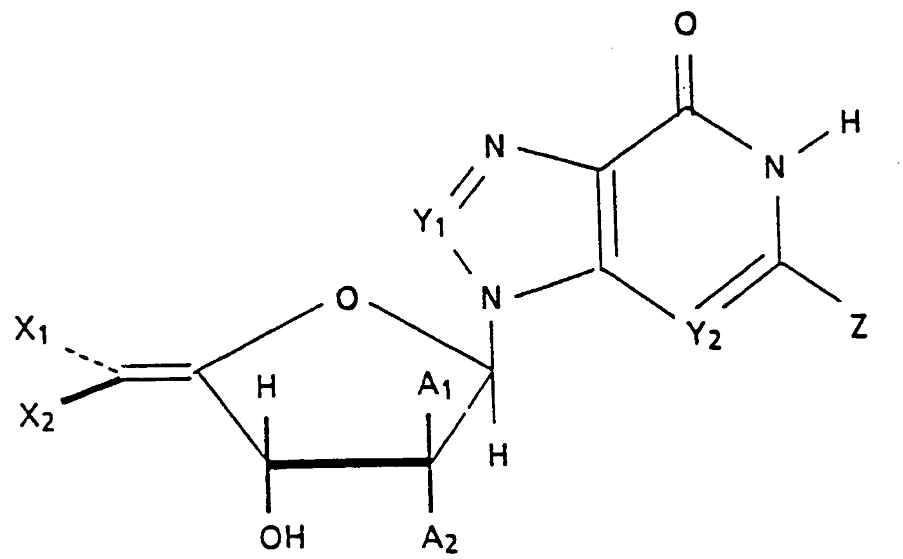

(1)

wherein $X_1$, $X_2$, $A_1$, $A_2$, $Y_1$, $Y_2$ and Z have the same meaning as in claim 1, comprising reacting a compound of the formula

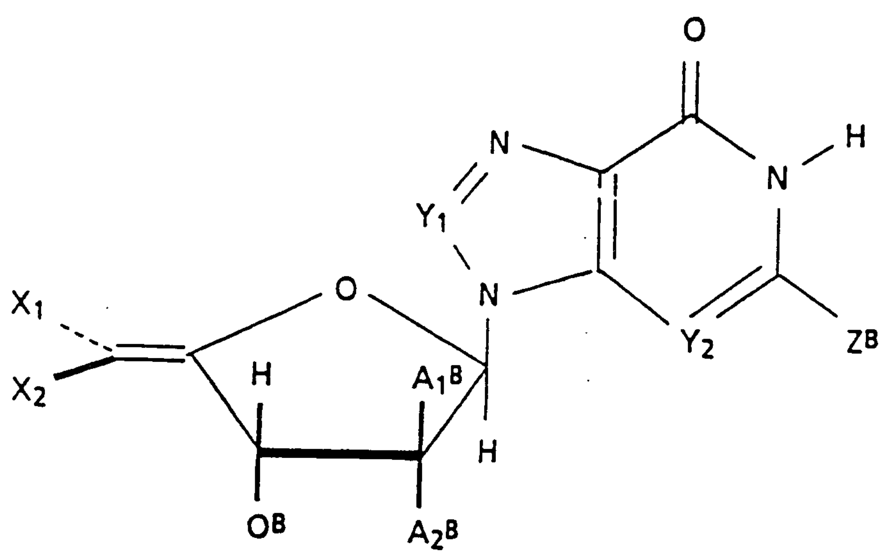

wherein $X_1$, $X_2$, $Y_1$, and $Y_2$ have the above meaning $O^B$, $A_1{}^B$ and $A_2{}^B$ represent OH, $A_1$ and $A_2$ groups, respectively, bearing hydroxy-blocking groups where appropriate, and $Z^B$ represents a Z group bearing an amino-blocking group where appropriate,

(a) with an acid to remove any hydroxy-protecting groups and

(b) with a base to remove any amino-protecting groups.

**14.** A method of inhibiting a purine nucleoside hydrolase in a protozoa comprising contacting said protozoa in vitro with an effective purine nucleoside hydrolase inhibitory amount of a compound of claim 1.

**15.** Use of a compound of any of claims 1 to 11 for the preparation of a pharmaceutical composition.

**16.** Use of a compound of any of claims 1 to 11 for the preparation of a pharmaceutical composition for treating a patient suffering from a protozoal infection.

17. Use according to claim 16, wherein the protozoal infection is by a protozoa of the genera Plasmodium, Trypanosoma, Leishmania, Trichomonas, Babesia, Toxoplasma, Pneumocystis or Theileria.

18. Use according to claim 16, wherein the patient is suffering from Ambiasis, Malaria, Leishmaniasis, Trypanosomiasis, Toxoplasmosis or a Pneumocysis carinii infection.

19. Use according to claim 16, wherein the patient is suffering from Chagas' Disease.

20. Use according to claim 16, wherein the patient is suffering from malaria.

21. A composition comprising an assayable amount of a compound of any of claims 1 to 11 in admixture with one or more inert carriers.

**Patentansprüche**

**Patentansprüche für folgende Vertragsstaaten : AT, BE, CH, DE, DK, FR, GB, IT, LI, LU, NL, SE**

1. Verbindung der Formel 1

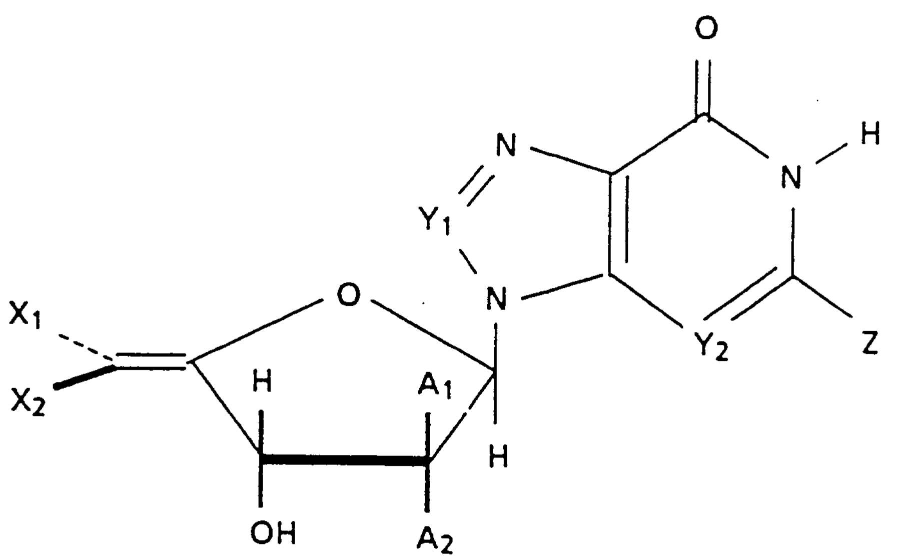

(1)

in der

| | |
|---|---|
| $X_1$ und $X_2$ | jeweils unabhängig Wasserstoff- oder Halogenatome bedeuten, mit der Maßgabe, daß mindestens einer der Reste $X_1$ und $X_2$ ein Halogenatom ist, |
| $A_1$ und $A_2$ | jeweils unabhängig Wasserstoffatome, Halogenatome oder eine Hydroxylgruppe darstellen, mit den Maßgaben, daß, falls $A_1$ eine Hydroxylgruppe ist, $A_2$ ein Wasserstoffatom bedeutet, und, falls $A_2$ eine Hydroxylgruppe ist, $A_1$ ein Wasserstoffatom darstellt, |
| $Y_1$ | ein Stickstoffatom, eine CH-Gruppe, eine CCl-Gruppe, eine CBr-Gruppe oder eine $CNH_2$-Gruppe ist, |
| $Y_2$ | ein Stickstoffatom oder eine CH-Gruppe ist, und |
| Z | ein Wasserstoffatom, ein Halogenatom oder eine $NH_2$-Gruppe darstellt. |

2. Verbindung nach Anspruch 1, in der $Y_1$ eine CH-Gruppe bedeutet.

3. Verbindung nach Anspruch 2, in der $Y_2$ eine NH-Gruppe bedeutet.

4. Verbindung nach Anspruch 3, in der $Y_2$ ein Stickstoffatom bedeutet.

5. Verbindung nach Anspruch 4, in der $A_1$ ein Wasserstoffatom und $A_2$ ein Hydroxylgruppe bedeuten.

6. Verbindung nach Anspruch 5, in der $X_1$ ein Halogenatom und $X_2$ ein Wasserstoffatom bedeuten.

7. Verbindung nach Anspruch 5, in der $X_1$ ein Wasserstoffatom und $X_2$ ein Halogenatom bedeuten.

8. Verbindung nach Anspruch 6, in der $X_1$ ein Chloratom bedeutet.

9. Verbindung nach Anspruch 7, in der $X_2$ ein Chloratom bedeutet.

10. Verbindung nach Anspruch 8 oder 9, in der Z ein Wasserstoffatom bedeutet.

11. Verbindung nach Anspruch 8 oder 9, in der Z eine $NH_2$-Gruppe bedeutet.

12. Verfahren zur Herstellung einer Verbindung der Formel 3

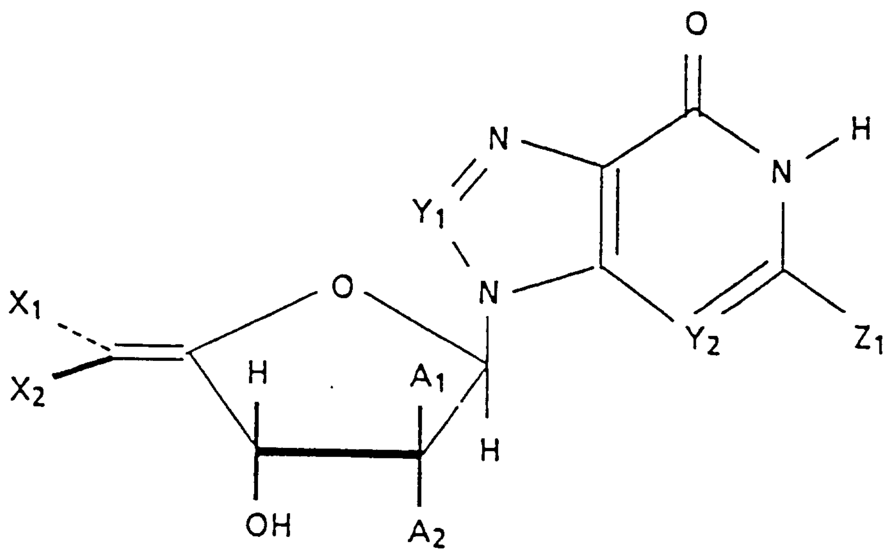

(3)

in der die Reste $X_1$, $X_2$, $A_1$, $A_2$, $Y_1$ und $Y_2$ dieselbe Bedeutung wie in Anspruch 1 haben, und $Z_1$ ein Wasserstoffatom oder ein Halogenatom bedeutet, umfassend die Umsetzung eines sauren Oxydationsmittels mit einer Verbindung der Formel 2

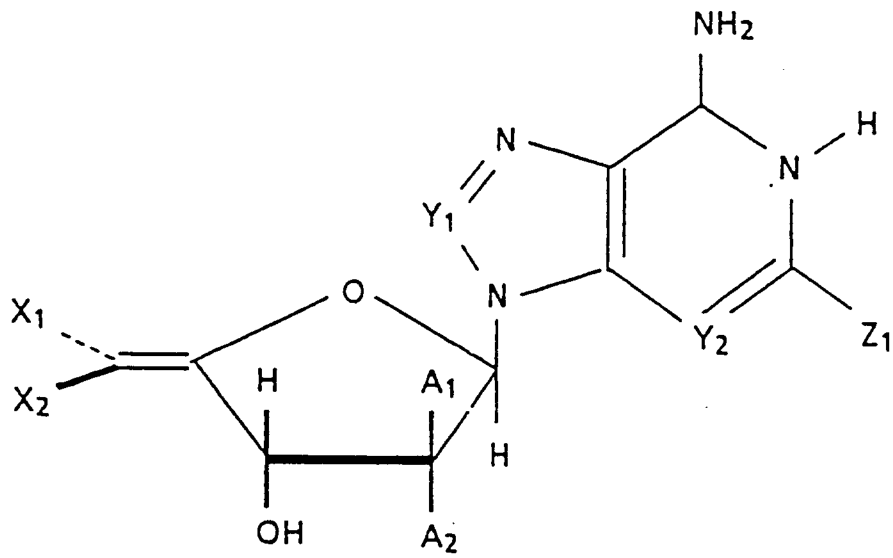

(2)

in der die Substituenten $X_1$, $X_2$, $A_1$, $A_2$, $Y_1$, $Y_2$ und $Z_1$ die vorstehend beschriebene Bedeutung haben.

**13.** Verfahren zur Herstellung einer Verbindung der Formel 1

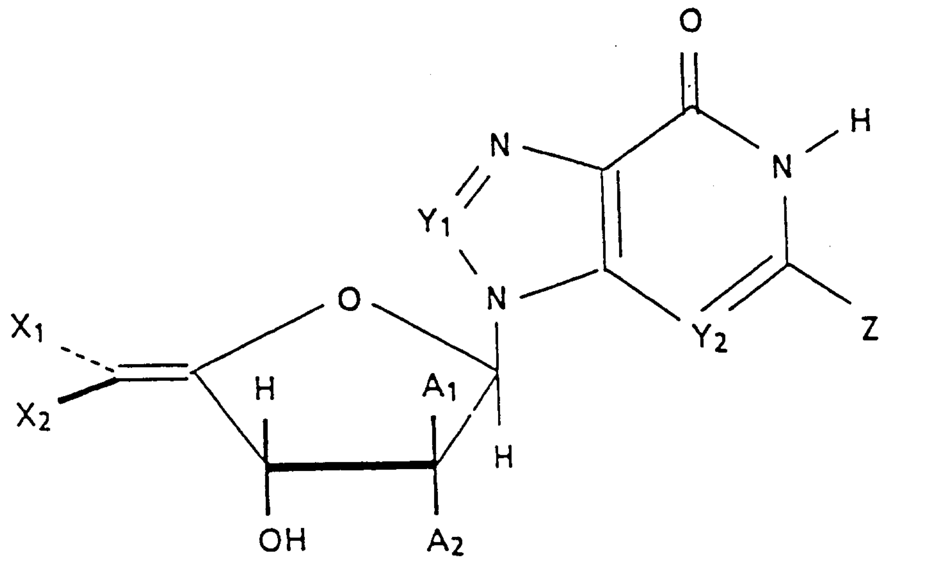

(1)

in der die Reste $X_1$, $X_2$, $A_1$, $A_2$, $Y_1$, $Y_2$ und Z dieselbe Bedeutung wie in Anspruch 1 haben, umfassend die Umsetzung einer Verbindung der Formel

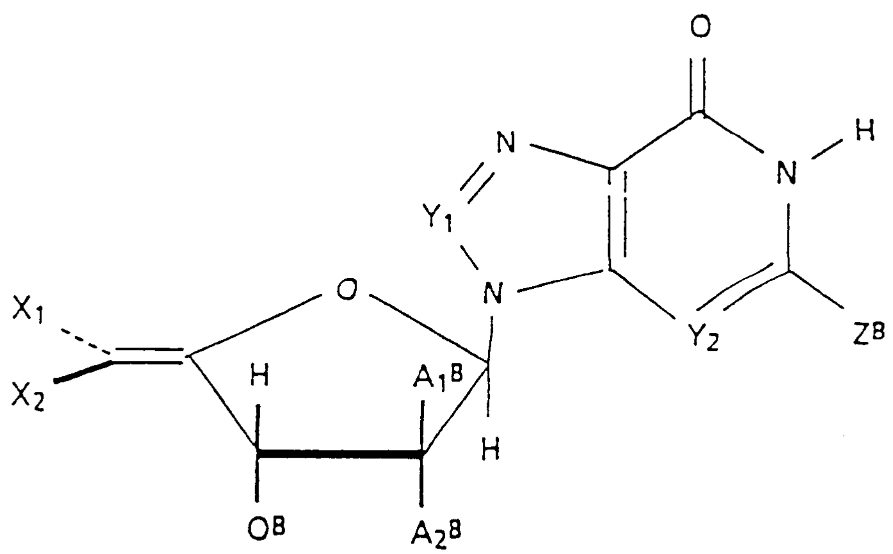

in der die Reste $X_1$, $X_2$, $Y_1$ und $Y_2$ die vorstehend beschriebene Bedeutung heben, $O^B$, $A_1{}^B$ und $A_2{}^B$ die Reste OH, $A_1$ bzw. $A_2$ bedeuten, die, falls angemessen, Hydroxylschutzgruppen tragen und $Z^B$ einen Rest Z bedeutet, der, falls angemessen, eine Aminoschutzgruppe trägt,

a) mit einer Säure, um irgendwelche Hydroxylschutzgruppen zu entfernen und

b) mit einer Base, um irgendwelche Aminoschutzgruppen zu entfernen.

**14.** Verfahren zur Inhibierung einer Purinnucleosid-Hydrolese in einem Protozoon, umfassend das in Kontakt bringen des Protozoon in vitro mit einer wirksamen, die Purinnucleosid-Hydrolase inhibierenden Menge einer Verbindung der Formel 1.

**15.** Verwendung einer Verbindung nach einem der Ansprüche 1 bis 11 zur Herstellung eines Arzneimittels.

**16.** Verwendung einer Verbindung nach einem der Ansprüche 1 bis 11 zur Herstellung eines Arzneimittels zur Behandlung eines Patienten, der an einer Protozoen-Infektion leidet.

17. Verwendung nach Anspruch 16, in der die Protozoen-Infektion durch ein Protozoon der Gattung Plasmodium, Trypanosoma, Leishmania, Trichomonas, Babesia, Toxoplasma, Pneumocystis oder Theileria geschieht.

18. Verwendung nach Anspruch 16, in der der Patient an einer Infektion durch Ambiasis, Malaria, Leishmaniasis, Trypanosomiasis, Toxoplasmosis oder Pneumocysis carinii leidet.

19. Verwendung nach Anspruch 16, in der der Patient an der Chagas-Krankheit leidet.

20. Verwendung nach Anspruch 16, in der der Patient an Malaria leidet.

21. Zusammensetzung, umfassend eine Probemenge einer Verbindung nach einem der Ansprüche 1 bis 11 in einer Beimischung mit einem oder mehreren inerten Trägern.

22. Arzneimittel, umfassend eine therapeutisch wirksame Menge einer Verbindung nach einem der Ansprüche 1 bis 11 in einer Beimischung mit einem oder mehreren pharmazeutisch verträglichen Trägern oder Exzipientien.

23. Arzneimittel nach Anspruch 22 zur Behandlung eines an einer Protozoen-Infektion leidenden Patienten.

**Patentansprüche für folgenden Vertragsstaat : ES**

1. Verfahren zur Herstellung einer Verbindung der Formel 1

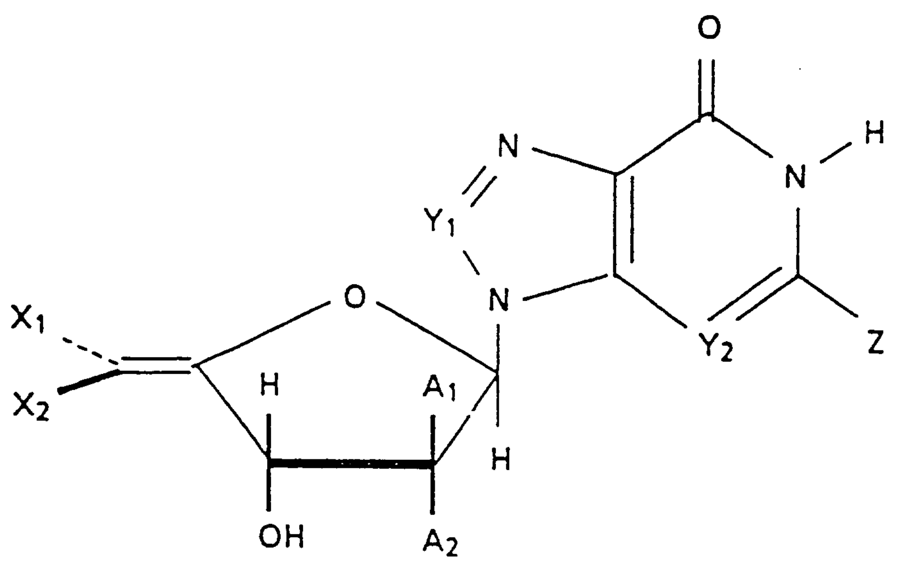

(1)

in der

| | |
|---|---|
| $X_1$ und $X_2$ | jeweils unabhängig Wasserstoff- oder Halogenatome bedeuten, mit der Maßgabe, daß mindestens einer der Reste $X_1$ und $X_2$ ein Halogenatom ist, |
| $A_1$ und $A_2$ | jeweils unabhängig Wasserstoffatome, Halogenatome oder eine Hydroxylgruppe darstellen, mit den Maßgaben, daß, falls $A_1$ eine Hydroxylgruppe ist, $A_2$ ein Wasserstoffatom bedeutet, und, falls $A_2$ eine Hydroxylgruppe ist, $A_1$ ein Wasserstoffatom darstellt, |
| $Y_1$ | ein Stickstoffatom, eine CH-Gruppe, eine CCl-Gruppe, eine CBr-Gruppe oder eine $CNH_2$-Gruppe ist, |
| $Y_2$ | ein Stickstoffatom oder eine CH-Gruppe ist, und |
| Z | ein Wasserstoffatom, ein Halogenatom oder eine $NH_2$-Gruppe darstellt, |

umfassend

1.1 bei der Herstellung von Verbindung 1, in der Z etwas anderes als eine $NH_2$-Gruppe bedeutet, Umsetzung eines Oxydationsmittels mit einer Verbindung der Formel 2

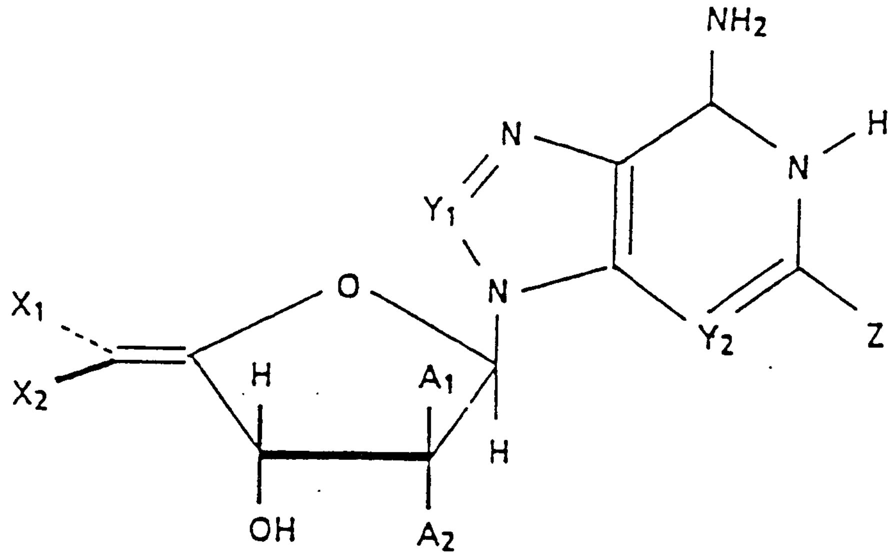

(2)

in der die Substituenten $X_1$, $X_2$, $A_1$, $A_2$, $Y_1$, $Y_2$ und Z die vorstehende Bedeutung haben; und
1.2 bei der Herstellung von Verbindung 1, in der Z ein Wasserstoffatom, ein Halogenatom oder eine $NH_2$-Gruppe bedeutet, Umsetzung einer Verbindung der Formel

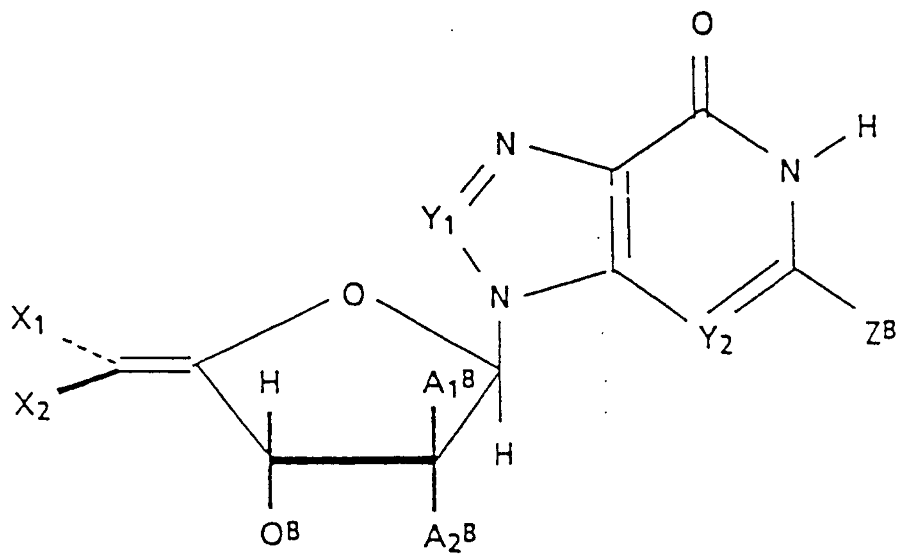

in der $O^B$, $A_1{}^B$ und $A_2{}^B$ die Reste OH, $A_1$ bzw. $A_2$ bedeuten, die, falls angemessen, Hydroxylschutzgruppen tragen und $Z^B$ einen Rest Z bedeutet, der,falls angemessen, eine Aminoschutzgruppe trägt,
(a) mit einer Säure, um irgendwelche Hydroxylschutzgruppen zu entfernen, und
(b) mit einer Base um irgendwelche Aminoschutzgruppen zu entfernen.

**2.** Verfahren nach Anspruch 1, bei dem $Y_1$ eine CH-Gruppe bedeutet.

**3.** Verfahren nach Anspruch 2, bei dem $Y_2$ eine NH-Gruppe bedeutet.

**4.** Verfahren nach Anspruch 3, bei dem $Y_2$ ein Stickstoffatom bedeutet.

**5.** Verfahren nach Anspruch 4, bei dem $A_1$ ein Wasserstoffatom und $A_2$ eine Hydroxylgruppe bedeuten.

**6.** Verfahren nach Anspruch 5, bei dem $X_1$ ein Halogenatom und $X_2$ ein Wasserstoffatom bedeuten.

**7.** Verfahren nach Anspruch 5, bei dem $X_1$ ein Wasserstoffatom und $X_2$ ein Halogenatom bedeuten.

8. Verfahren nach Anspruch 6, bei dem $X_1$ ein Chloratom bedeutet.

9. Verfahren nach Anspruch 7, bei dem $X_2$ ein Chloratom bedeutet.

10. Verfahren nach Anspruch 8 oder 9, bei dem Z ein Wasserstoffatom bedeutet.

11. Verfahren nach Anspruch 8 oder 9, bei den Z eine $NH_2$-Gruppe bedeutet.

12. Verfahren zur Inhibierung einer Purinnucleosid-Hydrolase in einem Protozoon, umfassend das in Kontakt bringen des Protozoon in vitro mit einer wirksamen, die Purinnuleosid-Hydrolase inhibierenden Menge einer nach dem Anspruch 1 hergestellten Verbindung.

13. Zusammensetzung, umfassend eine Probemenge einer nach einem der Ansprüche 1 bis 11 hergestellten Verbindung in Beimischung mit einem oder mehreren inerten Trägern.

**Patentansprüche für folgenden Vertragsstaat : GR**

1. Verbindung der Formel 1

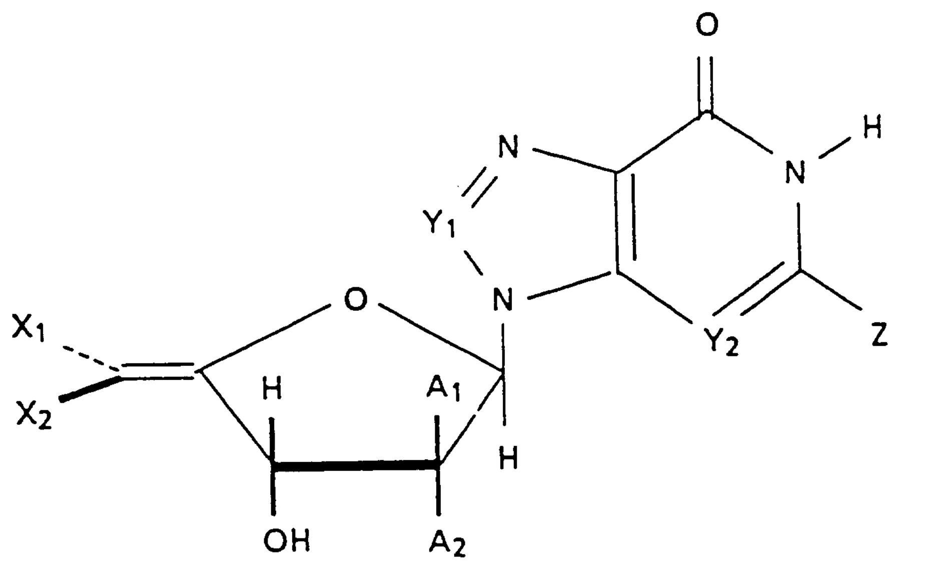

(1)

in der

| | |
|---|---|
| $X_1$ und $X_2$ | jeweils unabhängig Wasserstoff- oder Halogenatome bedeuten, mit der Maßgabe, daß mindestens einer der Reste $X_1$ und $X_2$ ein Halogenatom ist, |
| $A_1$ und $A_2$ | jeweils unabhängig Wasserstoffatome, Halogenatome oder eine Hydroxylgruppe darstellen, mit den Maßgaben, daß, falls $A_1$ eine Hydroxylgruppe ist, $A_2$ ein Wasserstoffatom bedeutet, und, falls $A_2$ eine Hydroxylgruppe ist, $A_1$ ein Wasserstoffatom darstellt, |
| $Y_1$ | ein Stickstoffatom, eine CH-Gruppe, eine CCl-Gruppe, eine CBr-Gruppe oder eine $CNH_2$-Gruppe ist, |
| $Y_2$ | ein Stickstoffatom oder eine CH-Gruppe ist, und |
| Z | ein Wasserstoffatom, ein Halogenatom oder eine $NH_2$-Gruppe darstellt. |

2. Verbindung nach Anspruch 1, in der $Y_1$ eine CH-Gruppe bedeutet.

3. Verbindung nach Anspruch 2, in der $Y_2$ eine NH-Gruppe bedeutet.

4. Verbindung nach Anspruch 3, in der $Y_2$ ein Stickstoffatom bedeutet.

5. Verbindung nach Anspruch 4, in der $A_1$ ein Wasserstoffatom und $A_2$ eine Hydroxylgruppe bedeuten.

6. Verbindung nach Anspruch 5, in dar $X_1$ ein Halogenatom und $X_2$ ein Wasserstoffatom bedeuten.

7. Verbindung nach Anspruch 5, in der $X_1$ ein Wasserstoffatom und $X_2$ ein Halogenatom bedeuten.

8. Verbindung nach Anspruch 6, in der $X_1$ ein Chloratom bedeutet.

9. Verbindung nach Anspruch 7, in der $X_2$ ein Chloratom bedeutet.

10. Verbindung nach Anspruch 8 oder 9, in der Z ein Wasserstoffatom bedeutet.

11. Verbindung nach Anspruch 8 oder 9, in der Z eine $NH_2$-Gruppe bedeutet.

12. Verfahren zur Herstellung einer Verbindung der Formel 3

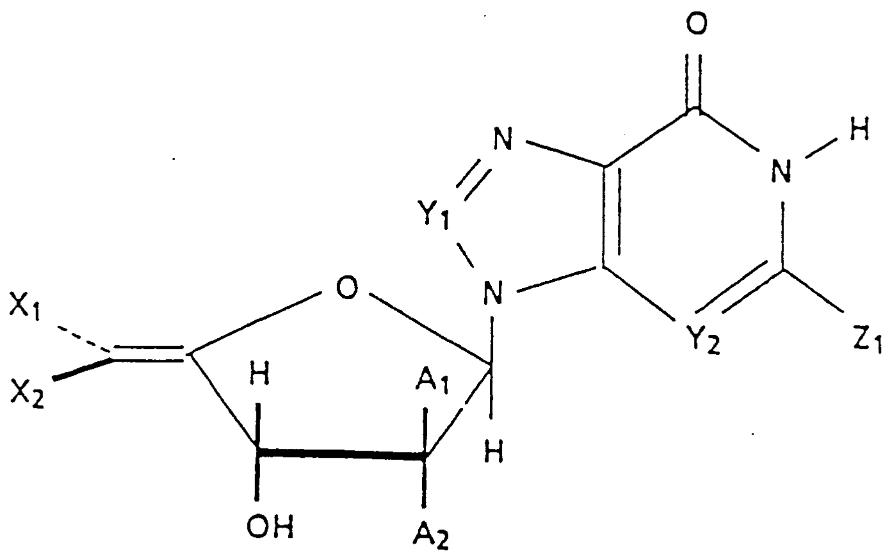

(3)

in der die Reste $X_1$, $X_2$, $A_1$, $A_2$, $Y_1$ und $Y_2$ dieselbe Bedeutung wie in Anspruch 1 haben, und $Z_1$ ein Wasserstoffatom oder ein Halogenatom bedeutet, umfassend die Umsetzung eines sauren Oxydationsmittels mit einer Verbindung der Formel 2

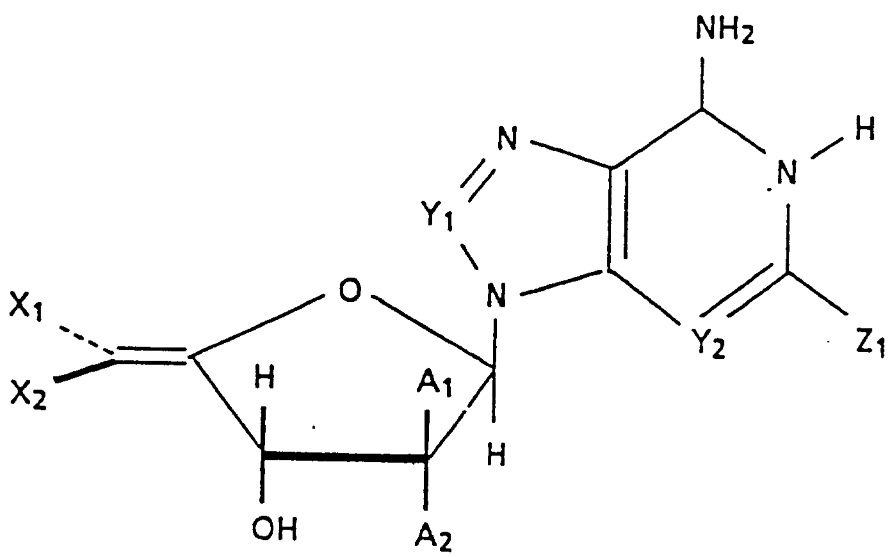

(2)

in der die Substituenten $X_1$, $X_2$, $A_1$, $A_2$, $Y_1$, $Y_2$ und $Z_1$ die vorstehend beschriebene Bedeutung haben.

**13.** Verfahren zur Herstellung einer Verbindung der Formel 1

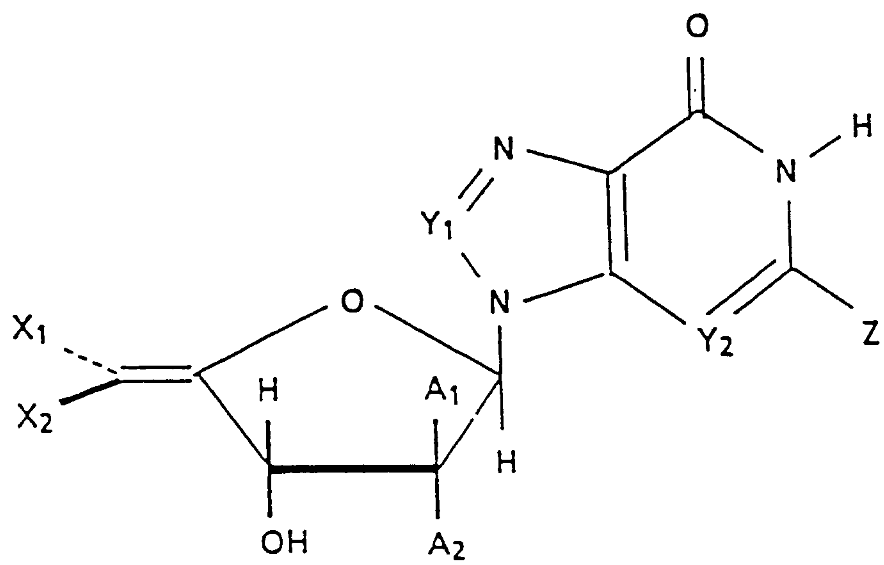

(1)

in der die Reste $X_1$, $X_2$, $A_1$, $A_2$, $Y_1$, $Y_2$ und Z dieselbe Bedeutung wie in Anspruch 1 haben, umfassend die Umsetzung einer Verbindung der Formel

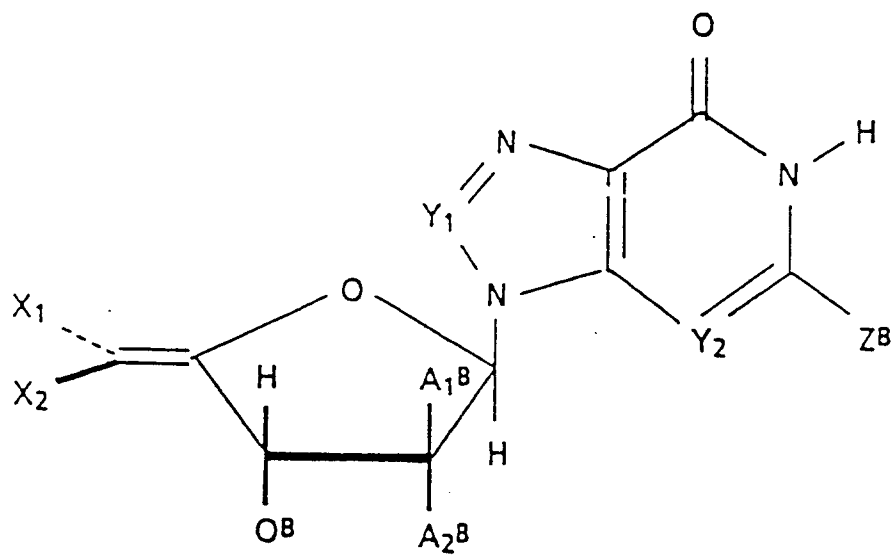

in der die Reste $X_1$, $X_2$, $Y_1$ und $Y_2$ die vorstehend beschriebene Bedeutung haben, $O^B$, $A_1{}^B$ und $A_2{}^B$ die Reste OH, $A_1$ bzw. $A_2$ bedeuten, die, falls angemessen, Hydroxylschutzgruppen tragen und $Z^B$ einen Rest Z bedeutet, der, falls angemessen, eine Aminoschutzgruppe trägt,

a) mit einer Säure, um irgendwelche Hydroxylschutzgruppen zu entfernen und

b) mit einer Base, um irgendwelche Aminoschutzgruppen zu entfernen.

**14.** Verfahren zur Inhibierung einer Purinnucleosid-Hydrolase in einem Protozoon, umfassend das in Kontakt bringen des Protozoon in vitro mit einer wirksamen, die Purinnucleosid-Hydrolase inhibierenden Menge einer Verbindung nach Anspruch 1.

**15.** Verwendung einer Verbindung nach einem der Ansprüche 1 bis 11 zur Herstellung eines Arzneimittels.

**16.** Verwendung einer Verbindung nach einem der Ansprüche 1 bis 11 zur Herstellung eines Arzneimittels zur Behandlung eines Patienten, der an einer Protozoen-Infektion leidet.

17. Verwendung nach Anspruch 16, in der die Protozoen-Infektion durch ein Protozoon der Gattung Plasmodium, Trypanosoma, Leishmania, Trichomonas, Babesia, Toxoplasma, Pneumocystis oder Theileria geschieht.

18. Verwendung nach Anspruch 16, in der der Patient an einer Infektion durch Ambiasis, Malaria, Leishmaniasis, Trypanosomiasis, Toxoplasmosis oder Pneumocysis carinii leidet.

19. Verwendung nach Anspruch 16, in der der Patient an der Chagas-Krankheit leidet.

20. Verwendung nach Anspruch 16, in der der Patient an Malaria leidet.

21. Zusammensetzung, umfassend eine Probemenge einer Verbindung nach einem der Ansprüche 1 bis 11 in einer Beimischung mit einem oder mehreren inerten Trägern.

**Revendications**

**Revendications pour les Etats contractants suivants : AT, BE, CH, DE, DK, FR, GB, IT, LI, LU, NL, SE**

1. Composé de formule 1 :

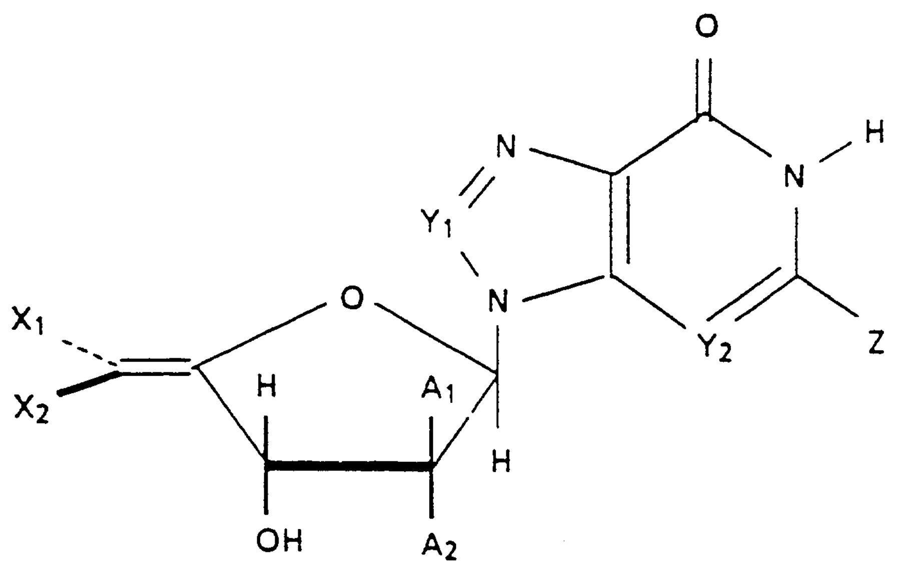

(1)

dans laquelle
$X_1$ et $X_2$ représentent chacun indépendamment l'hydrogène ou un halogène, à condition qu'au moins l'un des symboles $X_1$ et $X_2$ représente un atome d'halogène,
$A_1$ et $A_2$ représentent chacun indépendamment l'hydrogène, un halogène ou un hydroxyle, à condition que lorsque $A_1$ représente un hydroxyle, $A_2$ représente l'hydrogène et que lorsque $A_2$ représente un hydroxyle, $A_1$ représente l'hydrogène,
$Y_1$ représente l'azote, un coupe CH, un groupe CCl, un groupe CBr ou un groupe $CNH_2$,
$Y_2$ représente l'azote ou un groupe CH, et
Z représente l'hydrogène, un halogène ou $NH_2$.

2. Composé selon la revendication 1, dans lequel $Y_1$ représente un groupe CH.

3. Composé selon la revendication 2, dans lequel $Y_2$ représente un groupe NH.

4. Composé selon la revendication 3, dans lequel $Y_2$ représente l'azote.

5. Composé selon la revendication 4, dans lequel $A_1$ représente l'hydrogène et $A_2$ représente un hydroxyle.

6. Composé selon la revendication 5, dans lequel $X_1$ représente un halogène et $X_2$ représente l'hydrogène.

7. Composé selon la revendication 5, dans lequel $X_1$ représente l'hydrogène et $X_2$ représente un halogène.

8. Composé selon la revendication 6, dans lequel $X_1$ représente le chlore.

9. Composé selon la revendication 7, dans lequel $X_2$ représente le chlore.

10. Composé selon la revendication 8 ou 9, dans lequel Z représente l'hydrogène.

11. Composé selon la revendication 8 ou 9, dans lequel Z représente $NH_2$.

12. Procédé de préparation d'un composé de formule 3 :

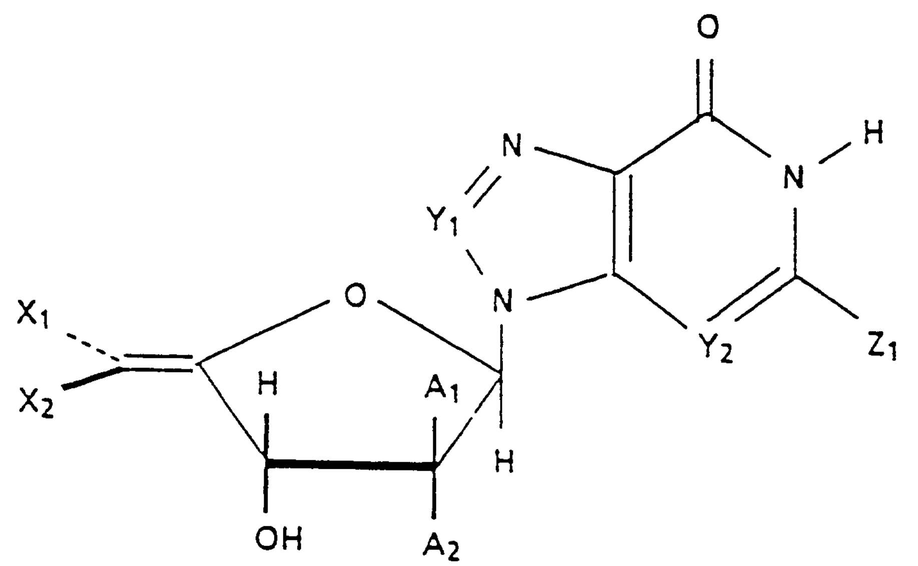

(3)

dans laquelle
$X_1$, $X_2$, $A_1$, $A_2$, $Y_1$ et $Y_2$ ont la même signification que dans la revendication 1, et
$Z_1$ représente l'hydrogène ou un halogène,
comprenant la réaction d'un agent oxydant acide avec un composé de formule 2 :

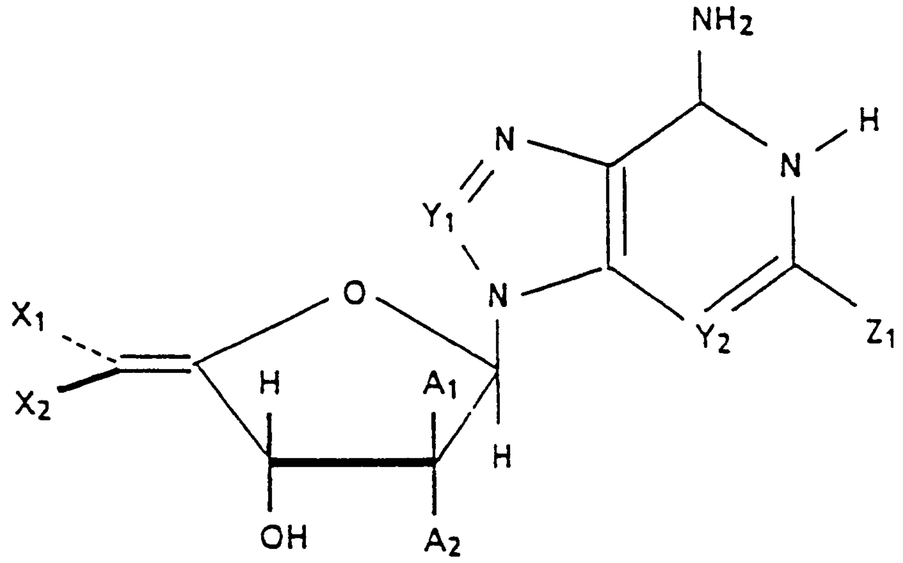

(2)

dans laquelle
les substituants $X_1$, $X_2$, $A_1$, $A_2$, $Y_1$, $Y_2$ et $Z_1$ ont la signification ci-dessus.

**13.** Procédé de préparation d'un composé de formule 1 :

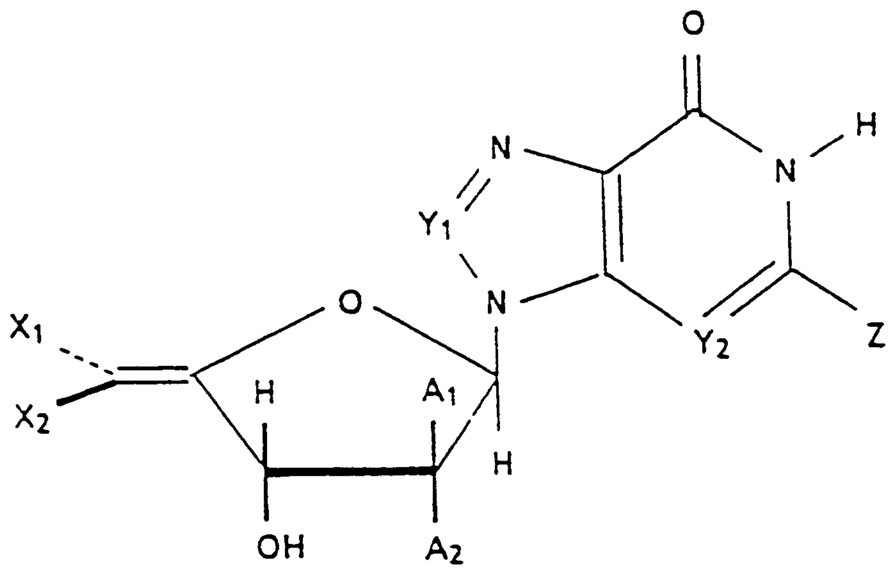

(1)

dans laquelle
$X_1$, $X_2$, $A_1$, $A_2$, $Y_1$, $Y_2$ et Z ont la même signification que dans la revendication 1, comprenant la réaction d'un composé de formule :

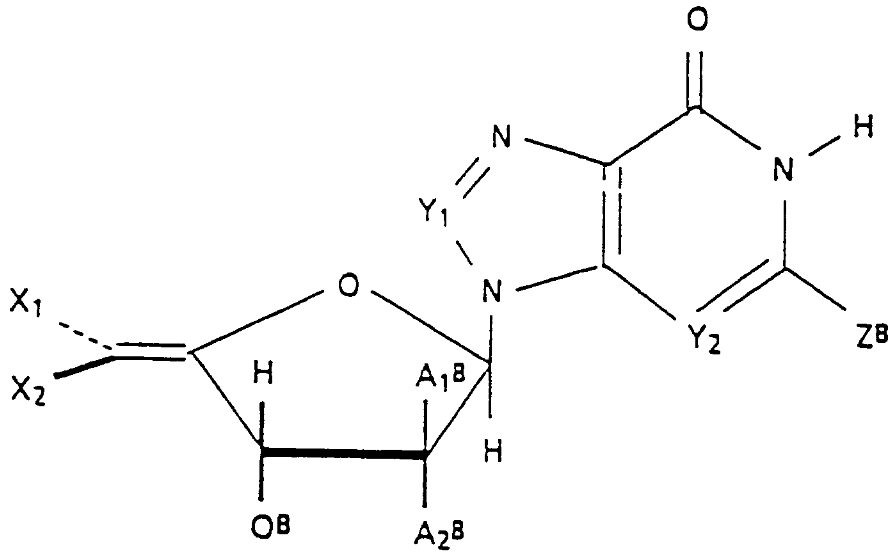

dans laquelle
$X_1$, $X_2$, $Y_1$ et $Y_2$ ont la signification ci-dessus, $O^B$, $A_1{}^B$ et $A_2{}^B$ représentent respectivement des groupes OH, $A_1$ et $A_2$ portant des groupes hydroxy-bloquants lorsque cela est approprié, et $Z^B$ représente un groupe Z portant un groupe amino-bloquant lorsque cela est approprié,

(a) avec un acide pour retirer tout groupe hydroxy-protecteur et

(b) avec une base pour retirer tout groupe amino-protecteur.

**14.** Procédé d'inhibition d'une nucléoside purique hydrolase chez un protozoaire, comprenant la mise en contact dudit protozoaire in vitro avec une quantité inhibitrice de nucléoside purique hydrolase efficace d'un composé selon la revendication 1.

**15.** Utilisation d'un composé selon l'une quelconque des revendications 1 à 11 pour la préparation d'une composition pharmaceutique.

**16.** Utilisation d'un composé selon l'une quelconque des revendications 1 à 11 pour la préparation d'une composition pharmaceutique pour traiter un patient souffrant d'une infection par un protozoaire.

**17.** Utilisation selon la revendication 16, dans laquelle l'infection par un protozoaire est due à un protozoaire des genres Plasmodium, Trypanosoma, Leishmania, Trichomonas, Babesia, Toxoplasma, Pneumocystis ou Theileria.

**18.** Utilisation selon la revendication 16, dans laquelle le patient souffre d'amibiase, de malaria, de leishmaniose, de trypanosomiase, de toxoplasmose ou d'une infection à Pneumocystis carinii.

**19.** Utilisation selon la revendication 16, dans laquelle le patient souffre de la maladie de Chagas.

**20.** Utilisation selon la revendication 16, dans laquelle le patient souffre de malaria.

**21.** Composition comprenant une quantité mesurable d'un composé selon l'une quelconque des revendications 1 à 11 en mélange avec un ou plusieurs véhicules inertes.

**22.** Composition pharmaceutique comprenant une quantité thérapeutiquement efficace d'un composé selon l'une quelconque des revendications 1 à 11 en mélange avec un ou plusieurs véhicules ou excipients pharmaceutiquement acceptables.

**23.** Composition pharmaceutique selon la revendication 22 pour traiter un patient souffrant d'une infection par un protozoaire.

**Revendications pour l'Etat contractant suivant : ES**

1. Procédé de préparation d'un composé de formule 1 :

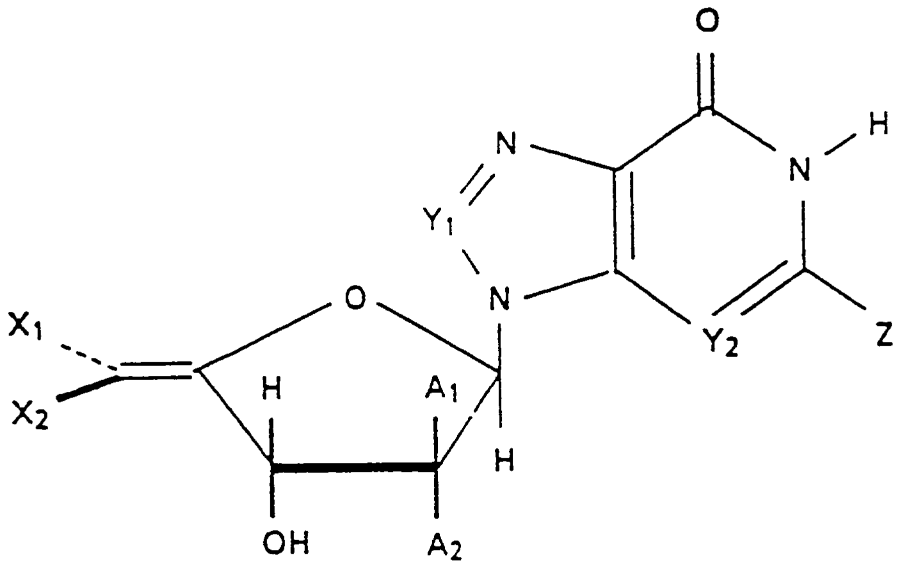

(1)

dans laquelle
$X_1$ et $X_2$ représentent chacun indépendamment l'hydrogène ou un halogène, à condition qu'au moins l'un des symboles $X_1$ et $X_2$ représente un atome d'halogène,
$A_1$ et $A_2$ représentent chacun indépendamment l'hydrogène, un halogène ou un hydroxyle, à condition que lorsque $A_1$ représente un hydroxyle, $A_2$ représente l'hydrogène et que lorsque $A_2$ représente un hydroxyle, $A_1$ représente l'hydrogène,
$Y_1$ représente l'azote, un groupe CH, un groupe CCl, un groupe CBr ou un groupe $CNH_2$,
$Y_2$ représente l'azote ou un groupe CH, et
Z représente l'hydrogène, un halogène ou $NH_2$, comprenant

1.1 lors de la préparation d'un composé 1 dans lequel Z ne représente pas $NH_2$, la réaction d'un agent oxydant avec un composé de formule 2 :

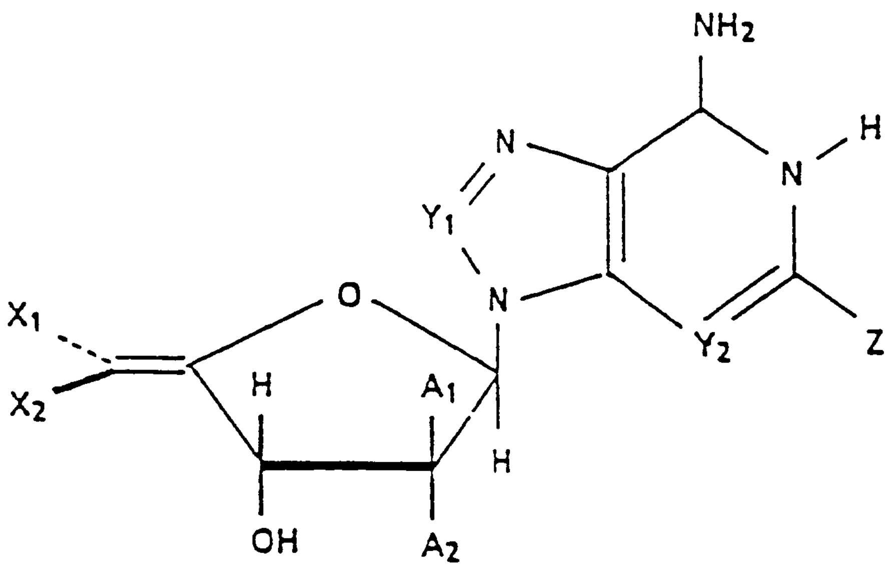

(2)

dans laquelle
les substituants $X_1$, $X_2$, $A_1$, $A_2$, $Y_1$, $Y_2$ et Z ont la signification ci-dessus; et

1.2 lors de la préparation d'un composé 1 dans lequel Z représente l'hydrogène, un halogène ou $NH_2$, la réaction d'un composé de formule :

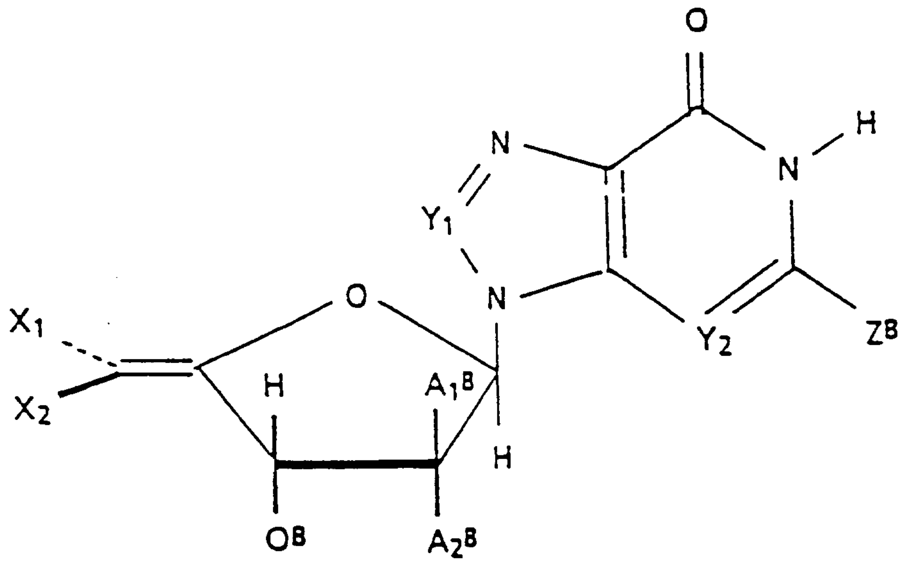

dans laquelle

$O^B$, $A_1{}^B$ et $A_2{}^B$ représentent respectivement des groupes OH, $A_1$ et $A_2$ portant des groupes hydroxy-bloquants lorsque cela est approprié, et $Z^B$ représente un groupe Z portant un groupe amino-bloquant lorsque cela est approprié,

(a) avec un acide pour retirer tout groupe hydroxy-protecteur

et

(b) avec une base pour retirer tout groupe amino-protecteur.

**2.** Procédé selon la revendication 1, dans lequel $Y_1$ représente un groupe CH.

**3.** Procédé selon la revendication 2, dans lequel $Y_2$ représente un groupe NH.

**4.** Procédé selon la revendication 3, dans lequel $Y_2$ représente l'azote.

**5.** Procédé selon la revendication 4, dans lequel $A_1$ représente l'hydrogène et $A_2$ représente un hydroxyle.

**6.** Procédé selon la revendication 5, dans lequel $X_1$ représente un halogène et $X_2$ représente l'hydrogène.

**7.** Procédé selon la revendication 5, dans lequel $X_1$ représente l'hydrogène et $X_2$ représente un halogène.

**8.** Procédé selon la revendication 6, dans lequel $X_1$ représente le chlore.

**9.** Procédé selon la revendication 7, dans lequel $X_2$ représente le chlore.

**10.** Procédé selon la revendication 8 ou 9, dans lequel Z représente l'hydrogène.

**11.** Procédé selon la revendication 8 ou 9, dans lequel Z représente $NH_2$.

**12.** Procédé d'inhibition d'une nucléoside purique hydrolase chez un protozoaire, comprenant la mise en contact dudit protozoaire in vitro avec une quantité inhibitrice de nucléoside purine hydrolase efficace d'un composé préparé selon la revendication 1.

**13.** Composition comprenant une quantité mesurable d'un composé préparé selon l'une quelconque des revendications 1 à 11 en mélange avec un ou plusieurs véhicules inertes.

**Revendications pour l'Etat contractant suivant : GR**

**1.** Composé de formule 1 :

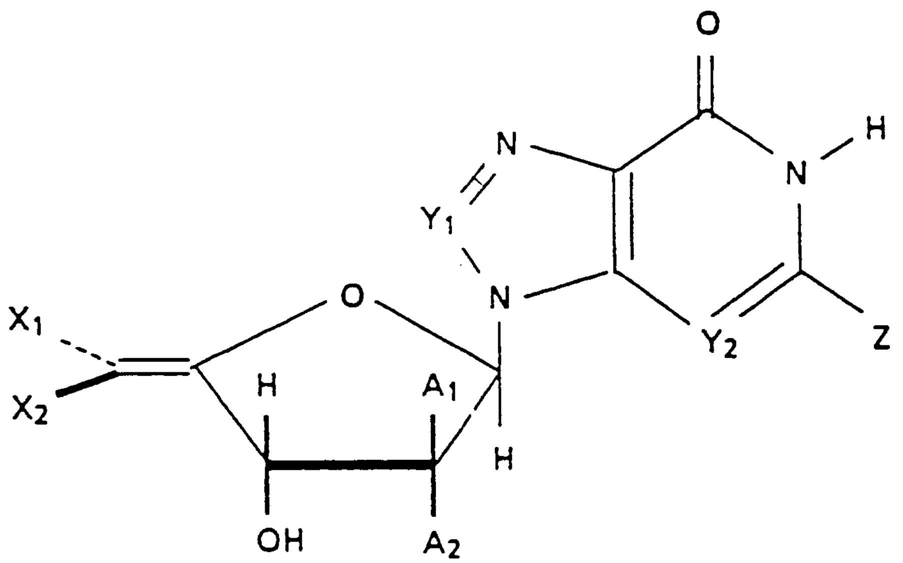

(1)

dans laquelle
$X_1$ et $X_2$ représentent chacun indépendamment l'hydrogène ou un halogène, à condition qu'au moins l'un des symboles $X_1$ et $X_2$ représente un atome d'halogène,
$A_1$ et $A_2$ représentent chacun indépendamment l'hydrogène, un halogène ou un hydroxyle, à condition que lorsque $A_1$ représente un hydroxyle, $A_2$ représente l'hydrogène et que lorsque $A_2$ représente un hydroxyle, $A_1$ représente l'hydrogène,
$Y_1$ représente l'azote, un groupe CH, un groupe CCl, un groupe CBr ou un groupe $CNH_2$,
$Y_2$ représente l'azote ou un groupe CH, et
Z représente l'hydrogène, un halogène ou $NH_2$.

**2.** Composé selon la revendication 1, dans lequel $Y_1$ représente un groupe CH.

**3.** Composé selon la revendication 2, dans lequel $Y_2$ représente un groupe NH.

**4.** Composé selon la revendication 3, dans lequel $Y_2$ représente l'azote.

**5.** Composé selon la revendication 4, dans lequel $A_1$ représente l'hydrogène et $A_2$ représente un hydroxyle.

**6.** Composé selon la revendication 5, dans lequel $X_1$ représente un halogène et $X_2$ représente l'hydrogène.

**7.** Composé selon la revendication 5, dans lequel $X_1$ représente l'hydrogène et $X_2$ représente un halogène.

**8.** Composé selon la revendication 6, dans lequel $X_1$ représente le chlore.

**9.** Composé selon la revendication 7, dans lequel $X_2$ représente le chlore.

**10.** Composé selon la revendication 8 ou 9, dans lequel Z représente l'hydrogène.

**11.** Composé selon la revendication 8 ou 9, dans lequel Z représente $NH_2$.

**12.** Procédé de préparation d'un composé de formule 3 :

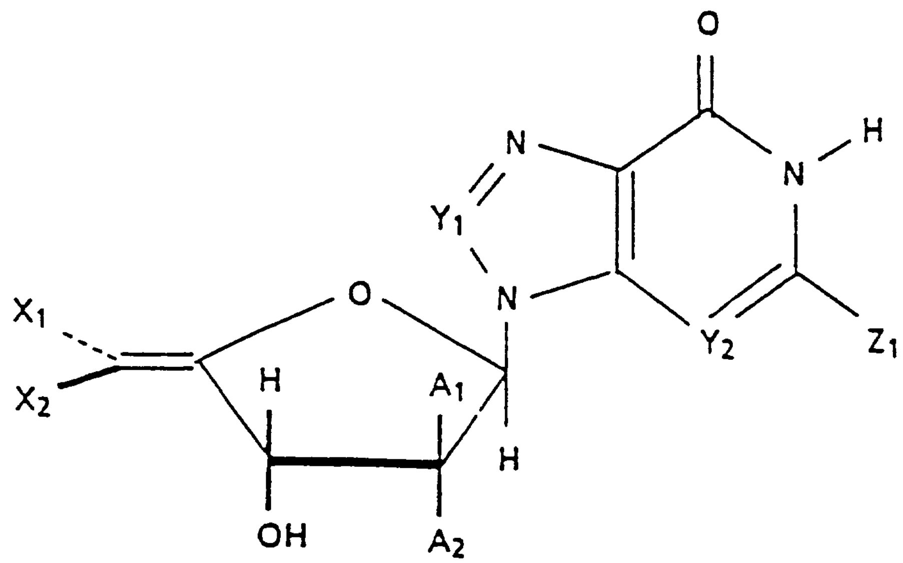

(3)

dans laquelle
$X_1$, $X_2$, $A_1$, $A_2$, $Y_1$ et $Y_2$ ont la même signification que dans la revendication 1, et $Z_1$ représente l'hydrogène ou un halogène,
comprenant la réaction d'un agent oxydant acide avec un composé de formule 2 :

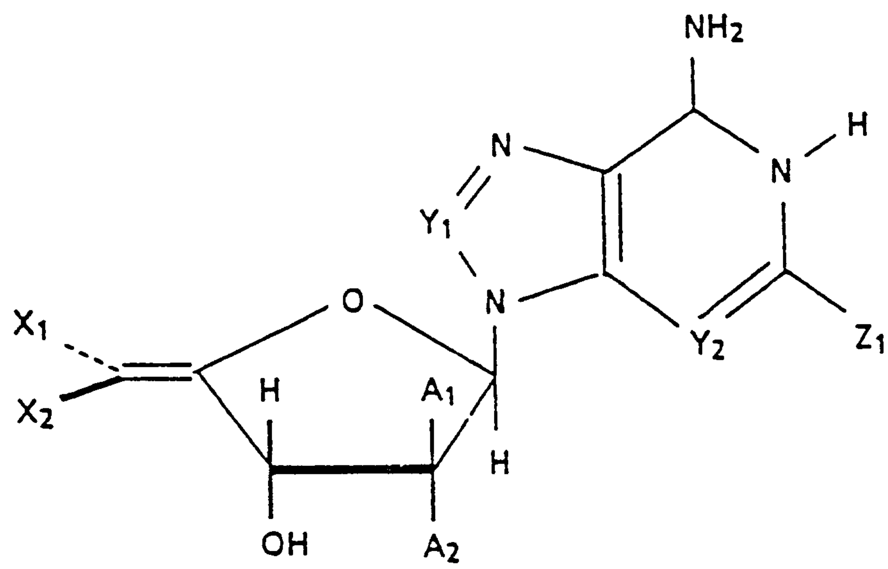

(2)

dans laquelle
les substituants $X_1$, $X_2$, $A_1$, $A_2$, $Y_1$, $Y_2$ et $Z_1$ ont la signification ci-dessus.

**13.** Procédé de préparation d'un composé de formule 1 :

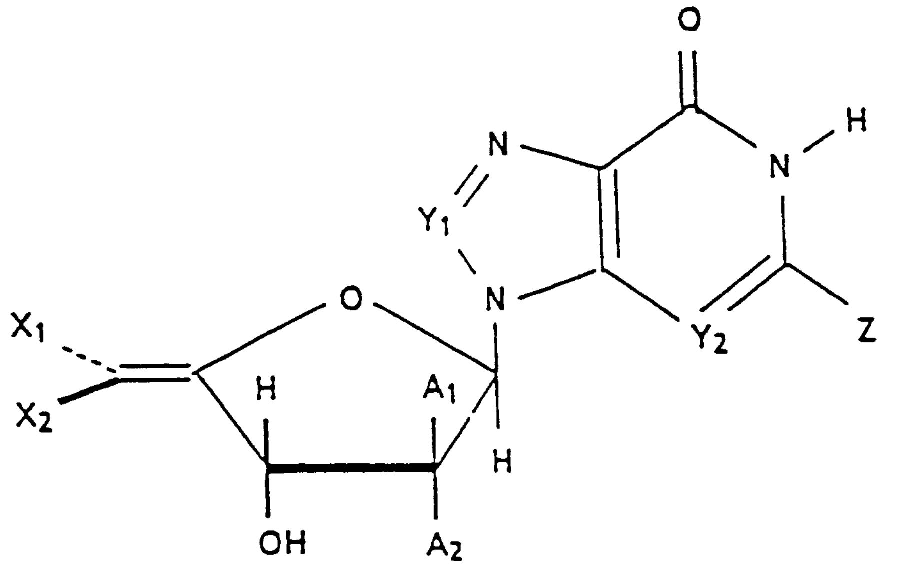

(1)

dans laquelle
$X_1$, $X_2$, $A_1$, $A_2$, $Y_1$, $Y_2$ et Z ont la même signification que dans la revendication 1, comprenant la réaction d'un composé de formule :

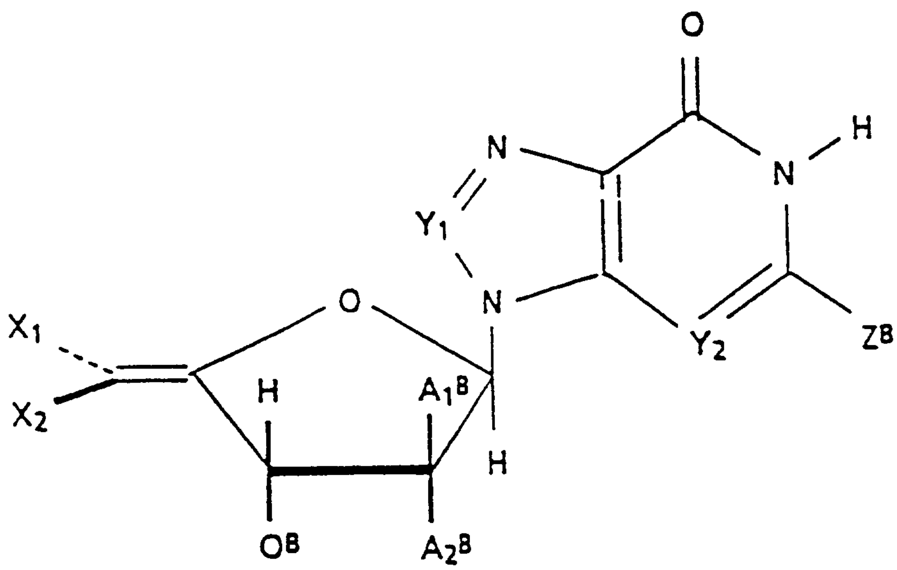

dans laquelle
$X_1$, $X_2$, $Y_1$ et $Y_2$ ont la signification ci-dessus, $O^B$, $A_1{}^B$ et $A_2{}^B$ représentent respectivement des groupes OH, $A_1$ et $A_2$ portant des groupes hydroxy-bloquants lorsque cela est approprié, et $Z^B$ représente un groupe Z portant un groupe amino-bloquant lorsque cela est approprié,
(a) avec un acide pour retirer tout groupe hydroxy-protecteur et
(b) avec une base pour retirer tout groupe amino-protecteur.

**14.** Procédé d'inhibition d'une nucléoside purique hydrolase chez un protozoaire, comprenant la mise en contact dudit protozoaire in vitro avec une quantité inhibitrice de nucléoside purique hydrolase efficace d'un composé selon la revendication 1.

**15.** Utilisation d'un composé selon l'une quelconque des revendications 1 à 11 pour la préparation d'une composition pharmaceutique.

**16.** Utilisation d'un composé selon l'une quelconque des revendications 1 à 11 pour la préparation d'une composition pharmaceutique pour traiter un patient souffrant d'une infection par un protozoaire.

**17.** Utilisation selon la revendication 16, dans laquelle l'infection par un protozoaire est due à un protozoaire des genres Plasmodium, Trypanosoma, Leishmania, Trichomonas, Babesia, Toxoplasma, Pneumocystis ou Theileria.

**18.** Utilisation selon la revendication 16, dans laquelle le patient souffre d'amibiase, de malaria, de leishmaniose, de trypanosomiase, de toxoplasmose ou d'une infection à Pneumocystis carinii.

**19.** Utilisation selon la revendication 16, dans laquelle le patient souffre de la maladie de Chagas.

**20.** Utilisation selon la revendication 16, dans laquelle le patient souffre de malaria.

**21.** Composition comprenant une quantité mesurable d'un composé selon l'une quelconque des revendications 1 à 11 en mélange avec un ou plusieurs véhicules inertes.